(19) Europäisches Patentamt
European Patent Office
Office européen des brevets

(11) **EP 4 101 479 A1**

(12) **EUROPEAN PATENT APPLICATION**
published in accordance with Art. 153(4) EPC

(43) Date of publication:
**14.12.2022 Bulletin 2022/50**

(21) Application number: **21750549.4**

(22) Date of filing: **03.02.2021**

(51) International Patent Classification (IPC):
*A61L 27/14* (2006.01)     *A61K 9/06* (2006.01)
*A61K 35/12* (2015.01)     *A61K 35/28* (2015.01)
*A61K 45/00* (2006.01)     *A61K 47/30* (2006.01)
*A61K 47/32* (2006.01)     *A61L 27/16* (2006.01)
*A61L 27/38* (2006.01)     *A61L 27/40* (2006.01)
*A61L 27/52* (2006.01)     *A61L 27/54* (2006.01)
*A61P 3/00* (2006.01)     *A61P 3/10* (2006.01)
*A61P 5/00* (2006.01)     *A61P 7/04* (2006.01)
*A61P 19/08* (2006.01)     *A61P 25/28* (2006.01)
*A61P 35/00* (2006.01)     *A61P 43/00* (2006.01)

(52) Cooperative Patent Classification (CPC):
**A61K 9/06; A61K 35/12; A61K 35/28; A61K 45/00;
A61K 47/30; A61K 47/32; A61L 27/14;
A61L 27/16; A61L 27/38; A61L 27/40; A61L 27/52;
A61L 27/54; A61P 3/00; A61P 3/10; A61P 5/00;**

(Cont.)

(86) International application number:
**PCT/JP2021/003991**

(87) International publication number:
**WO 2021/157626 (12.08.2021 Gazette 2021/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.02.2020 JP 2020019235**

(71) Applicants:
• **Shin-Etsu Chemical Co., Ltd.
Tokyo 100-0005 (JP)**
• **Japan Vam & Poval Co., Ltd.
Sakai-shi, Osaka 592-8331 (JP)**

• **Tohoku University
Sendai-shi, Miyagi 980-8577 (JP)**

(72) Inventors:
• **KANESATO Shuhei
Tokyo 100-0005 (JP)**
• **KIMURA Yoshihiro
Sakai-shi, Osaka 592-8331 (JP)**
• **OHARUDA Akinobu
Sakai-shi, Osaka 592-8331 (JP)**
• **GOTO Masafumi
Sendai-shi, Miyagi 980-8577 (JP)**

(74) Representative: **Lederer & Keller Patentanwälte
Partnerschaft mbB
Unsöldstraße 2
80538 München (DE)**

(54) **ANGIOGENESIS DEVICE**

(57)     The present invention provides a novel angiogenesis device. The angiogenesis device comprises an angiogenic component and a polymer, and is used to induce angiogenesis at a site for implantation of a cell- or tissue-containing device. The polymer may comprise, for example, at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

Fig. 1

(52) Cooperative Patent Classification (CPC): (Cont.)
     **A61P 7/04; A61P 19/08; A61P 25/28;**
     **A61P 35/00; A61P 43/00**

**Description**

TECHNICAL FIELD

The present invention relates to an angiogenesis device and others.

BACKGROUND ART

Cell- or tissue-containing devices, e.g., cell- or tissue-embedding devices (collectively simply called cell-containing devices or cell-embedding devices) have been studied for implantation therapy. Cell- or tissue-embedding devices are capable of holding live cells or tissue to supply hormones, proteins or other physiologically active substances involved in metabolic functions to a patient or to detoxify harmful substances in a patient body, thereby serving as an alternative organ in a human or animal patient suffering from a disease to prevent and/or treat the disease. Cell- or tissue-embedding devices are advantageous in various aspects. For example, cell- or tissue-embedding devices may have an immunoisolation layer capable of protecting live cells or tissue from the immune defense system in the recipient. Cell- or tissue-embedding devices having such an immunoisolation layer do not require administration of immunosuppressive drugs, in contrast to living donor organ transplantation, and therefore can avoid adverse side effects associated with immunosuppressive drugs. Cell- or tissue-embedding devices can also be implanted into a patient body in a less invasive manner. Lastly, cell- or tissue-embedding devices can solve the problem of organ donor shortage.

Cell- or tissue-embedding devices are currently available in various forms, such as those in the form of a microcapsule or a macrocapsule formulation that encapsulates live cells or tissue in a polymer (e.g., a cell formulation). These types of cell- or tissue-embedding devices have strongly cross-linked polymer structure that is capable of protecting the cells or tissue from the immune defense system in the recipient. The cell- or tissue-embedding devices also utilizes the molecular permeability of the polymer to supply hormones or other substances secreted from the cells or tissue to the body of the recipient.

Several cell-embedding devices are proposed in literature. For example, Patent literature 1 discloses a cell- or tissue-embedding device having an aqueous gel serving as an immunoisolation layer, the aqueous gel containing a modified polyvinyl alcohol resin having an active carbonyl group (A) and a crosslinking agent (B). Patent literature 2 discloses a cell- or tissue-embedding device having an aqueous gel serving as an immunoisolation layer, the aqueous gel containing a polyvinyl alcohol resin having a triad syndiotacticity of 32 to 40% (A) .

CITATION LIST

PATENT LITERATURE

    Patent literature 1: WO 2018/155621
    Patent literature 2: WO 2018/155622

SUMMARY OF INVENTION

TECHNICAL PROBLEM

An object of the present invention is to provide a novel angiogenesis device and others.

The inventors investigated the currently available cell-embedding devices as described above and found that the cell-embedding devices may fail to supply sufficient oxygen or nutrients to the live cells or tissue within the device. Live cells or tissue not supplied with enough oxygen or nutrients may cause central necrosis, and may severely deteriorate the functions of the cell-embedding devices.

Such a problem often occurs when the devices are implanted in subcutaneous regions where blood vessels are not abundant. More severe shortage of oxygen or nutrients in subcutaneous regions will occur in large animals having thicker subcutaneous tissue.

Based on the findings, the inventors conducted extensive studies and further found that the above problems can be solved by inducing angiogenesis at a site for implantation of a cell-embedding device before the implantation of the cell-embedding device. The inventors made further studies and completed the present invention.

SOLUTION TO PROBLEM

That is, the present invention relates to the following.

(1) An angiogenesis device for inducing or promoting angiogenesis (or a device for inducing angiogenesis, or a device for promoting angiogenesis) at a site for implantation of a cell- or tissue-containing device (e.g., a cell- or tissue-embedding device) before implantation of the cell- or tissue-containing device, wherein the angiogenesis device comprises an angiogenic component (an angiogenesis-promoting component) and a polymer.

(2) An angiogenesis device (or a device for inducing angiogenesis, or a device for promoting angiogenesis) adapted to be used in combination with a cell- or tissue-containing device (e.g., a cell- or tissue-embedding device), wherein the angiogenesis device comprises an angiogenic component (an angiogenesis-promoting component) and a polymer.

(3) A combination device, a device, a kit or a combination, comprising an angiogenesis device and a cell- or tissue-containing device (e.g., a cell- or tissue-embedding device), wherein the angiogenesis device comprises an angiogenic component (an angiogenesis-promoting component) and a polymer, wherein the angiogenesis device may be a device for inducing angiogenesis, a device for promoting angiogenesis, or an angiogenesis device for inducing angiogenesis at a site for implantation of a cell- or tissue-containing device (e.g., a cell- or tissue-embedding device) before implantation of the cell- or tissue-containing device, and wherein the cell- or tissue-containing device may be a cell- or tissue-containing device (e.g., a cell- or tissue-embedding device) to be implanted into a site where angiogenesis has been induced by the angiogenesis device.

(4) The device according to any one of the above (1) to (3), wherein the angiogenic component comprises mesenchymal stem cells.

(5) The device according to any one of the above (1) to (4), wherein the angiogenic component comprises adipose-derived stem cells.

(6) The device according to any one of the above (1) to (5), wherein the polymer comprises a polyvinyl alcohol resin (A).

(7) The device according to any one of the above (1) to (6), wherein the polymer comprises at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

(8) The device according to any one of the above (1) to (7), wherein the polymer comprises at least one or more polyvinyl alcohol resins (A) selected from a modified polyvinyl alcohol resin having an active carbonyl group (A1), and a polyvinyl alcohol resin having a triad syndiotacticity of 32 to 40% (A2) .

(9) The device according to the above (7) or (8), wherein the polyvinyl alcohol resin (A1) comprises a diacetone acrylamide-modified polyvinyl alcohol.

(10) The device according to the above (9), wherein the diacetone acrylamide-modified polyvinyl alcohol contains 0.5 to 15 mol% diacetone acrylamide units.

(11) The device according to any one of the above (7) to (10), wherein the polyvinyl alcohol resin (A2) has a degree of saponification of 90 mol% or more and a degree of polymerization of 100 to 10,000.

(12) The device according to any one of the above (7) to (11), wherein the polyvinyl alcohol resin (A2) is a saponified product of a polymer containing vinyl pivalate as a polymerization component, and wherein the polyvinyl alcohol resin (A2) contains vinyl pivalate units.

(13) The device according to any one of the above (1) to (12), wherein the polymer is in the form of an aqueous gel.

(14) The device according to any one of the above (1) to (13), wherein the polymer is in the form of an aqueous gel with a stress of 0.1 kPa or more, e.g., 0.5 to 100 kPa, at 20°C.

(15) The device according to any one of the above (1) to (14), wherein the polymer is in the form of an aqueous gel with a rate of volume change of 10% or more as determined by immersing the gel in water for one month.

(16) The device according to any one of the above (1) to (15), wherein the polymer is in the form of an aqueous gel with a stress of 0.3 kPa or more, e.g., 0.3 to 90 kPa, at 20°C and a rate of volume change of 20% or more, e.g., 20 to 90%, as determined by immersing the gel in water for one month.

(17) The device according to any one of the above (13) to (16), wherein the aqueous gel further comprises a crosslinking agent.

(18) The device according to any one of the above (13) to (17), wherein the aqueous gel comprises at least one crosslinking agent selected from hydrazide compounds and semicarbazide compounds.

(19) The device according to any one of the above (1) to (18), wherein the angiogenesis device further comprises a cell culture component.

(20) The device according to the above (19), wherein the cell culture component contains at least one buffer solution selected from an acetate buffer solution and a phosphate buffer solution.

(21) The device according to any one of the above (1) to (20), wherein the angiogenic component is present within the polymer.

(22) The device according to any one of the above (1) to (21), wherein the angiogenic component is dissolved or dispersed in the polymer.

(23) The device according to any one of the above (1) to (22), wherein the polymer forms a scaffold and/or an adhesion preventive layer.

(24) The device according to any one of the above (1) to (23), wherein the device is used for subcutaneous implantation.

(25) The device according to any one of the above (1) to (24), wherein the cell- or tissue-containing device (e.g., a cell- or tissue-embedding device) has an immunoisolation layer comprising the polyvinyl alcohol resin (A).

(26) The device according to any one of the above (1) to (25), wherein the cell- or tissue-containing device (e.g., a cell- or tissue-embedding device) has an immunoisolation layer comprising at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

(27) An implantation method in a human or a non-human animal, the method comprising implanting a cell- or tissue-containing device (e.g., a cell- or tissue-embedding device) into a site in which the angiogenesis device according to any one of the above (1) to (26) has been implanted and subsequently retrieved.

(28) A method for preventing and/or treating and/or improving a disease or a symptom in a human or a non-human animal, the method comprising implanting a cell- or tissue-containing device (e.g., a cell- or tissue-embedding device) into a site in which the angiogenesis device according to any one of the above (1) to (26) has been implanted and subsequently retrieved, wherein the cell or tissue may contribute to prevention, treatment and/or improvement of the disease or the symptom.

(29) The method according to the above (27) or (28), further comprising implanting the angiogenesis device to induce angiogenesis.

(30) The method according to any one of the above (27) to (29), wherein the angiogenesis device is retrieved without causing bleeding, inflammation and/or rupture of a blood vessel (a newly formed blood vessel), and wherein the site for implantation of the cell- or tissue-containing device has no bleeding, inflammation and/or rupture of a blood vessel.

(31) The method according to any one of the above (27) to (30), wherein the device is implanted subcutaneously.

ADVANTAGEOUS EFFECTS OF INVENTION

The present invention provides a novel angiogenesis device. The angiogenesis device is suitable for various applications involving angiogenesis. The angiogenesis device is especially suitable as an angiogenesis device adapted to be used in combination with a cell- or tissue-containing device (e.g., a cell- or tissue-embedding device). In particular, the angiogenesis device is suitable as a device for inducing angiogenesis at a site for implantation of a cell-embedding device before the implantation of the cell-embedding device.

Another aspect of the present invention provides an angiogenesis device capable of efficiently inducing angiogenesis.

Another aspect of the present invention provides an angiogenesis device that may be implanted into a site and subsequently retrieved from the site to create a space with newly formed blood vessels, in particular, a space in which newly formed blood vessels spread, which may serve as an implantation pocket.

Such a pocket with newly formed blood vessels can be created at a site with a small number of blood vessels, such as a subcutaneous site, which makes the angiogenesis device very useful. In this manner, the angiogenesis device is capable of efficiently inducing angiogenesis at a site for implantation of a cell-embedding device, thereby greatly enhancing the functions of the cell-embedding device. The angiogenesis device according to this aspect of the invention makes it possible to avoid situations where live cells or tissue is implanted before angiogenesis proceeds and may efficiently inhibit or prevent central necrosis.

Another aspect of the present invention provides an angiogenesis device that may be efficiently retrieved after implantation. The angiogenesis device, for example, rarely forms adhesions to the surrounding tissue, and when being retrieved, rarely causes damage to the surrounding tissue or the vascular bed formed by the angiogenesis device. In this manner, the angiogenesis device is capable of preventing or reducing bleeding, inflammation (induction of inflammation) or release of exudates from the implantation site. The angiogenesis device according to this aspect of the invention is adapted to induce angiogenesis and to facilitate the retrieval of the angiogenesis device, and is thus very useful.

The inventors studied and found that even when angiogenesis is induced by the angiogenesis device, bleeding or release of exudates may deteriorate the functions of the cell-embedding device to be implanted into the site in which angiogenesis has been induced. The angiogenesis device according to the above aspects of the invention is capable of preventing or reducing bleeding, inflammation or release of exudates and much more efficiently enhancing the functions of a separately implanted cell-embedding device.

BRIEF DESCRIPTION OF DRAWINGS

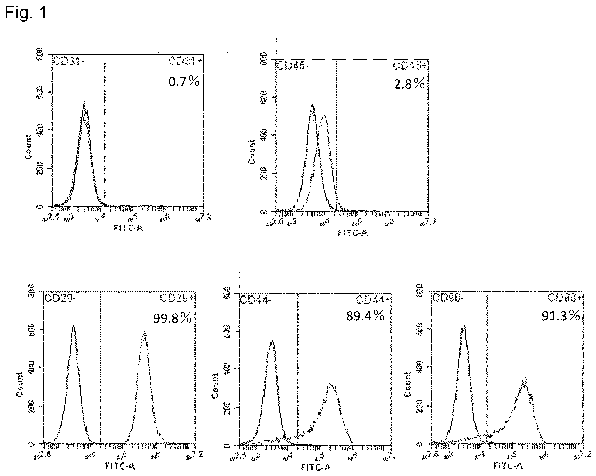

Fig. 1 shows charts showing the results of flow cytometry analysis of isolated adipose-derived stem cells (ADSCs) with labeled markers.

DESCRIPTION OF EMBODIMENTS

Angiogenesis device

The angiogenesis device of the present invention comprises at least an angiogenic component and a polymer.

Angiogenic component

The angiogenic component, i.e., a component capable of promoting angiogenesis may be any component that contributes to or promotes angiogenesis. Examples of such a component include cell growth factors, such as vascular endothelial growth factor (VEGF), fibroblast growth factor (FGF) and platelet-derived growth factor (PDGF) ; placental growth factor (PIGF); and cells, such as cells capable of secreting cell growth factors.
Examples of the cells include stem cells such as mesenchymal stem cells (MSCs), including, for example, adipose-derived stem cells (ADSCs), marrow-derived stem cells (BMSCs), placenta-derived stem cells, and umbilical cord-derived stem cells.
The cells are preferably mesenchymal stem cells, more preferably adipose-derived stem cells (ADSCs).
The angiogenic component may be derived from any organisms, and is preferably derived from, for example, mammals such as humans, monkeys, pigs, rats, mice, dogs and cats.
The angiogenic component may be added as it is to the device, e.g., cells serving as the angiogenic component may be directly added to the device. Alternatively, the cells may be grown into spheroids or loaded on a support and then added to the device. Preferably, the cells (not grown into spheroids or other shapes) are directly added to the device to avoid complicated handling or prevent reduction in the viability of the angiogenic component (e.g., MSCs).
The angiogenic component may be a commercially available product including a genetically modified product, or may be isolated from a subject (such as a mammal) having an angiogenic component.
The angiogenic component can be isolated from a subject by a known method or a conventional technique. For example, MSCs (e.g., ADSCs) may be isolated as follows.
The subcutaneous adipose tissue is harvested from the abdominal and inguinal regions of 7- to 8-week-old male Lewis rats (Japan SLC) and washed with phosphate buffer solution. The adipose tissue is minced, and enzymatically treated in Hanks buffer solution containing 1 mg/mL Type II collagenase (Sigma-Aldrich) and 1 mmol/L $CaCl_2 \cdot 2H_2O$ under shaking at 100 rpm at 37°C for 20 minutes. Remaining undigested adipose tissue is further treated with the enzyme for additional 20 minutes. The cell and tissue suspension is filtered through a nylon mesh of 70 $\mu$m to remove the residual tissue fragments. The filtrate is centrifuged at 350 $\times$ g at 20°C for 5 minutes to fractionate into a precipitate fraction containing stem cells and a mature adipocyte fraction mainly composed of lipids. The precipitate fraction is washed with Hanks buffer solution, and suspended in serum-containing medium for adipose-derived stem cells (ADSC-1, Kohjin Bio) containing 100 units/mL penicillin-100 $\mu$g/mL streptomycin (Thermo Fisher Scientific) at 5 $\times$ 10$^6$ cells/mL. Ten milliliters of the suspension is transferred to a T75 flask (culture area: 75 cm$^2$), and primary culture is performed under 5% $CO_2$ at a humidity of 95% at 37°C. The medium is replaced on days 1, 2 and 4 of culture, and non-adherent cells are removed. Cells in the flask are cultured until reaching 50 to 80% confluency. The primary cells are detached from the flask, dissociated into single cells and stored by freezing. The dissociation into single cells is performed by removing the medium from the flask, washing the cells with phosphate buffer solution (PBS), adding 3 mL of 0.05% Trypsin-EDTA (Thermo Fisher Scientific) and incubating at 37°C for 3 minutes to detach the cells from the surface of the flask. The isolated adipose-derived stem cells (ADSCs) are suspended in cryopreservative medium CELLBANKER 1plus (Nippon Zenyaku Kogyo) at 1 $\times$ 10$^6$ cells/mL, and frozen at -80°C and stored in liquid nitrogen. Frozen cells are then thawed, and suspended in serum-containing medium for ADSCs containing 100 units/mL penicillin-100 $\mu$g/mL streptomycin at 3.3 $\times$ 10$^4$ cells/mL. Ten milliliters of the suspension is transferred to a T75 flask, and the cells are cultured under 5% $CO_2$ at a humidity of 95% at 37°C until reaching 50 to 80% confluency. The second passage cells are detached from the flask, and dissociated into single cells (in the same manner as in the trypsin-EDTA treatment before storing by freezing).
The presence of the cells of interest can be confirmed by, for example, reacting the isolated cells with a fluorescence-conjugated antibody against a cell surface marker, and detecting the fluorescence with a flow cytometer to determine the positive percentage of the marker.

An exemplary flow cytometry analysis was conducted as follows. The cells were treated with 0.05% trypsin-EDTA and detached from the flask. The collected cells were washed with PBS, and $1 \times 10^5$ cells were suspended in 100 μL of PBS containing 0.5% bovine serum albumin and 2 mmol/L EDTA. The cells were reacted with CD29-FITC antibody, CD44-FITC antibody, CD45-FITC antibody, CD90-FITC antibody (BD Biosciences) or CD31-FITC antibody (Gene Tex), and analyzed with a flow cytometer (BD Accuri™ C6, BD Biosciences). The results demonstrate that ADSCs at the second passage isolated from the adipose tissue of Lewis rats expressed positive markers for ADSCs: 99.8% of the cells expressed CD29, 89.4% of the cells expressed CD44, and 91.3% of the cells expressed CD90. CD31 and CD45, which are negative markers for ADSCs, were only 0.7% and 2.8%, respectively (Fig. 1).

The angiogenic component may be a single type or a combination of two or more types.

Polymer

The angiogenesis device comprises a polymer.

The polymer contained in the angiogenesis device may be in the form of an aqueous gel (hydrogel), and may contain the angiogenic component dissolved or dispersed therein as described later.

The polymer may form or serve as a scaffold (supporting framework) and/or an adhesion preventive layer in the angiogenesis device.

The polymer is selected to be suitable for the arrangement, configuration or other requirements in the angiogenesis device. The polymer may be selected from, for example, structural proteins such as collagen, elastin and keratin; gelatin; glycosaminoglycans such as hyaluronic acid, chondroitin sulfate, keratan sulfate, dermatan sulfate, heparan sulfate and heparin; proteoglycans; cell adhesion molecules such as fibronectin, laminin, fibrinogen and vitronectin; fibrin; fibroin; sericin; alginic acid; chitosan; agarose; cellulose; cellulose derivatives such as cellulose nanofibers, carboxymethyl cellulose, and hydroxypropyl cellulose; synthetic polypeptides; polylactic acid; polyglycolic acid; polycaprolactone; polyethylene glycol; polypropylene glycol; 2-methacryloyloxyethyl phosphorylcholine; poly(meth)acrylic acid; polyacrylamide; poly-N-isopropylacrylamide; dextrin; silicone; polyurethane; fluororesins such as polytetrafluoroethylene and perfluoroalkoxyalkane; polyvinyl alcohol resins; and others.

The polymer may be a hydrophilic polymer or a water-soluble polymer. The hydrophilic polymer or the water-soluble polymer contained in the angiogenesis device may typically be converted into a water-insoluble form, e.g., a gel, by crosslinking or other techniques.

The polymer may be a single type or a combination of two or more types.

A polyvinyl alcohol resin is particularly preferred among the polymers as exemplified above. Accordingly, the polymer may contain at least a polyvinyl alcohol resin.

The polyvinyl alcohol resin will be described in detail below.

Polyvinyl alcohol resin (A)

The polyvinyl alcohol resin may typically be a saponified product of a polymer containing at least a vinyl ester monomer as a polymerization component. Such a polyvinyl alcohol resin may be any polyvinyl alcohol resin containing vinyl alcohol units, and may contain vinyl ester units or structural units derived from a vinyl ester monomer, e.g., structural units derived from a fatty acid vinyl ester, such as vinyl acetate units and vinyl pivalate units (described later) and/or may contain other units, e.g., structural units derived from an unsaturated monomer having an active carbonyl group (described later) or other structural units derived from other unsaturated monomers.

The viscosity of a 4% by mass aqueous solution of the polyvinyl alcohol resin (at 20°C) may be, for example, but is not limited to, 1 mPa·s or more, 2 mPa·s or more, 3 mPa·s or more, 5 mPa·s or more, etc., or may be, for example, 800 mPa·s or less, 500 mPa·s or less, 300 mPa·s or less, 200 mPa·s or less, 150 mPa·s or less, 100 mPa·s or less, 80 mPa·s or less, etc.

Typically, a polyvinyl alcohol resin having a viscosity of about 3 to 300 mPa·s as measured as a 4% by mass aqueous solution may be used as a suitable polyvinyl alcohol resin.

The viscosity of a 4% by mass aqueous solution of a polyvinyl alcohol resin may be measured in accordance with, for example, JIS K 6726.

The polyvinyl alcohol resin used in the invention can be selected in terms of its type, composition, etc., and may be, but is not limited to, a completely saponified polyvinyl alcohol resin (e.g., having a degree of saponification of 97 mol% or more), or a partially saponified polyvinyl alcohol resin (e.g., having a degree of saponification of less than 97 mol%).

The (average) degree of saponification may be measured in accordance with, for example, JIS K 6726.

In particular, a polyvinyl alcohol resin suitable as the polyvinyl alcohol resin (A) may be at least one or more polyvinyl alcohol resins selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3), or especially at least one polyvinyl alcohol resin selected from the resins (A1) and (A2).

These resins (A1) to (A3) will be described in detail below.

Modified polyvinyl alcohol resin having active carbonyl group (A1)

The modified polyvinyl alcohol resin having an active carbonyl group (A1) (simply called the modified PVA resin herein) may be, for example, a modified PVA copolymer produced by copolymerization of a fatty acid vinyl ester with an unsaturated monomer having an active carbonyl group followed by saponification of the resulting copolymer, or a post-modified PVA produced by directly contacting a conventionally produced PVA or modified PVA resin with a compound having an active carbonyl group, such as liquid diketene or diketene gas. Amodified PVA copolymer is preferred for better stability and safety of the PVA resin and for better workability in the gelation process.

The fatty acid vinyl ester used in the production of the modified PVA copolymer may be, for example, but is not limited to, vinyl formate, vinyl acetate, vinyl propionate, vinyl pivalate, etc., and vinyl acetate is industrially preferred. These fatty acid vinyl esters may be subjected to a conventionally known polymerization method, such as bulk polymerization, solution polymerization, suspension polymerization, and emulsion polymerization. Solution polymerization using an alcoholic solvent, such as methanol, is industrially preferred.

The unsaturated monomer having an active carbonyl group may be, for example, but is not limited to, diacetone acrylamide, diacetone methacrylamide, diacetone acrylate, diacetone methacrylate, acetoacetoxy acrylamide, acetoacetoxy methacrylamide, etc. These unsaturated monomers may be used alone or in combination of two or more. Diacetone acrylamide is industrially preferred. The modified PVA copolymer is preferably diacetone acrylamide-modified PVA.

The copolymerization reaction between the fatty acid vinyl ester and the unsaturated monomer having an active carbonyl group according to the present invention may further include an additional unsaturated monomer capable of copolymerizing with the fatty acid vinyl ester and the unsaturated monomer having an active carbonyl group to the extent that the additional unsaturated monomer does not impair the effects of the invention.

The additional unsaturated monomer may be one or more unsaturated monomers selected from carboxyl group-containing unsaturated monomers, such as (meth) acrylic acid, maleic acid, maleic anhydride, fumaric acid, crotonic acid, itaconic acid, and undecylenic acid; unsaturated dibasic acid monoalkyl esters, such as monomethyl maleate and monomethyl itaconate; amide group-containing unsaturated monomers, such as acrylamide, dimethylacrylamide, dimethylaminoethylacrylamide, diethylacrylamide, dimethylaminopropylacrylamide, isopropylacrylamide, N-methylolacrylamide, and N-vinylacetamide; vinyl halides, such as vinyl chloride and vinyl fluoride; glycidyl group-containing unsaturated monomers, such as allyl glycidyl ether and glycidyl methacrylate; lactam group-containing unsaturated monomers including, e.g., N-vinylpyrrolidones such as N-vinyl-2-pyrrolidone and N-vinyl-alkylpyrrolidone (e.g., N-vinyl-mono- or di-$C_{1-4}$ alkyl-pyrrolidones, such as N-vinyl-3-propyl-2-pyrrolidone, N-vinyl-5-methyl-2-pyrrolidone, N-vinyl-5-ethyl-2-pyrrolidone, N-vinyl-5,5-dimethyl-2-pyrrolidone, and N-vinyl-3,5-dimethyl-2-pyrrolidone), N-allylpyrrolidones such as N-allyl-2-pyrrolidone, N-vinylpiperidones such as N-vinyl-2-piperidone and N-vinyl-alkylpiperidone (e.g., N-vinyl-mono- or di-$C_{1-4}$ alkyl-piperidones, such as N-vinyl-6-methyl-2-piperidone and N-vinyl-6-ethyl-2-piperidone), and N-vinylcaprolactams such as N-vinyl-$\varepsilon$-caprolactam and N-vinyl-alkylcaprolactam (e.g., N-vinyl-mono- or di-$C_{1-4}$ alkyl-caprolactams, such as N-vinyl-7-methyl-2-caprolactam and N-vinyl-7-ethyl-2-caprolactam); alkyl vinyl ethers such as $C_{1-20}$ alkyl vinyl ethers (e.g., methyl vinyl ether, n-propyl vinyl ether, i-propylvinyl ether, n-butyl vinyl ether, i-butyl vinyl ether, t-butyl vinyl ether, lauryl vinyl ether, dodecyl vinyl ether, and stearyl vinyl ether); nitriles such as acrylonitrile and methacrylonitrile; hydroxy group-containing unsaturated monomers, including, e.g., $C_{1-20}$ monoalkyl allyl alcohols such as allyl alcohol and isopropenyl allyl alcohol, $C_{1-20}$ dialkyl allyl alcohols such as dimethyl allyl alcohol, and hydroxy $C_{1-20}$ alkyl vinyl ethers such as hydroxy ethyl vinyl ether and hydroxy butyl vinyl ether; acetyl group-containing unsaturated monomers, including, e.g., $C_{1-20}$ alkyl allyl acetates such as allyl acetate, dimethylallyl acetate, and isopropenylallyl acetate; (meth)acrylic acid esters, including, e.g., (meth)acrylic acid alkyl esters, such as (meth) acrylic acid $C_{1-20}$ alkyl esters such as methyl (meth)acrylate, ethyl (meth)acrylate, 2-ethylhexyl acrylate, and n-butyl acrylate; vinylsilanes such as trimethoxyvinylsilane, tributylvinylsilane, and diphenylmethylvinylsilane; polyoxyalkylene (meth)acrylates, such as polyoxyethylene (meth)acrylate and polyoxypropylene (meth)acrylate; polyoxyalkylene (meth)acrylamides, such as polyoxyethylene (meth)acrylamide and polyoxypropylene (meth)acrylamide; polyoxyalkylene vinyl ethers, such as polyoxyethylene vinyl ether and polyoxypropylene vinyl ether; polyoxyalkylene alkylvinyl ethers, such as polyoxyethylene allyl ether, polyoxypropylene allyl ether, polyoxyethylene butylvinyl ether, and polyoxypropylene butylvinyl ether; $\alpha$-olefins such as ethylene, propylene, n-butene, and 1-hexene; butenes such as 3,4-dihydroxy-1-butene, 3,4-diacyloxy-1-butene, 3-acyloxy-4-hydroxy-1-butene, 4-acyloxy-3-hydroxy-1-butene, and 3,4-diacyloxy-2-methyl-1-butene; pentenes such as 4,5-dihydroxy-1-pentene, 4,5-diacyloxy-1-pentene, 4,5-dihydroxy-3-methyl-1-pentene, and 4,5-diacyloxy-3-methyl-1-pentene; hexenes such as 5,6-dihydroxy-1-hexene and 5,6-diacyloxy-1-hexene; unsaturated amine monomers, such as N,N-dimethylallylamine, N-allylpiperazine, 3-piperidine acrylic acid ethyl ester, 2-vinylpyridine, 4-vinylpyridine, 2-methyl-6-vinylpyridine, 5-ethyl-2-vinylpyridine, 5-butenylpyridine, 4-pentenylpyridine, and 2-(4-pyridyl)allyl alcohol; quaternary ammonium compound-containing unsaturated monomers, such as dimethylaminoethyl acrylate methyl chloride quaternary salt, N,N-dimethylaminopropyl acrylamide methyl chloride quaternary salt, and N,N-dimethylaminopropyl acrylamide methyl benzenesulfonate quaternary salt; aromatic unsaturated monomers such as styrene; sulfonic acid group-containing unsaturated monomers, such as 2-acrylamide-2-methylpropanesulfonic acid or its alkali metal salts, ammonium salts or organic amine salts, 2-acrylamide-1-methylpropanesulfonic acid or its alkali metal salts, ammonium salts or organic amine salts, 2-methacrylamide-2-methylpropanesulfonic acid or its alkali metal salts, ammonium salts or organic amine salts, vinyl sulfonic acid or its alkali metal salts, ammonium salts or organic amine salts, allyl sulfonic acid or its alkali metal salts, ammonium salts or organic amine salts, and methallyl sulfonic acid or its alkali metal salts, ammonium salts or organic amine salts; glycerol monoallyl ether; 2,3-diacetoxy-1-allyloxypropane; 2-acetoxy-1-allyloxy-3-hydroxypropane; 3-acetoxy-1-allyloxy-3-hydroxypropane; 3-acetoxy-1-allyloxy-2-hydroxypropane; glycerol monovinyl ether; glycerol monoisopropenyl ether; acryloyl morpholine; vinyl ethylene carbonate; vinylimidazole; and vinylcarbazole.

The amount of the additional unsaturated monomer may be, for example, but is not limited to, 10 mol or less relative to 100 mol of the vinyl ester monomers.

The produced modified PVA copolymer may be post-modified by acetalization, urethanation, etherification, grafting, phosphorylation, acetoacetylation, cationization, or other reactions in accordance with a known method to the extent that the effects of the present invention are not impaired.

A polymerization catalyst is used in the production of the modified PVA copolymer, and may typically be, but is not limited to, an azo compound or a peroxide.

An organic acid, such as tartaric acid, citric acid, and acetic acid, may be added to the polymerization reaction to prevent the hydrolysis of the fatty acid vinyl ester.

A polymerization terminator may be used to terminate the polymerization. The polymerization terminator may be, for example, but is not limited to, m-dinitrobenzene etc.

The shape of a polymerization vessel, the type of a polymerization agitator, the polymerization temperature, the pressure in the polymerization vessel, or other conditions in the copolymerization of the fatty acid vinyl ester and the unsaturated monomer having an active carbonyl group according to the present invention may be in accordance with a conventionally known method.

The copolymer of the fatty acid vinyl ester and the unsaturated monomer having an active carbonyl group may be saponified by any conventionally known method according to the present invention. For example, a conventionally known alcoholysis or hydrolysis is applicable using a basic catalyst, such as sodium hydroxide, potassium hydroxide, and sodium methoxide, or an acidic catalyst, such as hydrochloric acid, sulfuric acid, and p-toluenesulfonic acid.

The solvent used in the saponification reaction may be, for example, an alcohol such as methanol and ethanol; an ester such as methyl acetate; a ketone such as acetone and methyl ethyl ketone; an aromatic hydrocarbon such as benzene and toluene; tetrahydrofuran; etc. These solvents may be used alone or in combination of two or more. The temperature, duration, and other conditions of the saponification reaction are not particularly limited.

The drying, grinding or washing of the saponified product may be done by any methods and may be performed by a conventionally known method.

When the modified PVA resin is a PVA modified with the unsaturated monomer having an active carbonyl group (e.g., diacetone acrylamide), the amount of the unsaturated monomer units having an active carbonyl group (e.g., diacetone acrylamide units) contained in the modified PVA resin is, for example, 0.5 to 20 mol%, preferably 0.5 to 15 mol%, more preferably 1 to 12 mol%, and further preferably 2 to 10 mol% (e.g., 3 to 8 mol%) relative to the all the structural units in the modified PVA resin (relative to the total amount of the structural monomers).

The amount of the unsaturated monomer units having an active carbonyl group (e.g., diacetone acrylamide units) contained in the modified PVA resin is preferably 0.5 mol% or more, and the modified PVA resin containing such an amount of the unsaturated monomer units may have many reactive sites with a crosslinking agent and have sufficient strength (stress) as an angiogenesis device. The amount of the unsaturated monomer units contained in the modified PVA resin is preferably 20 mol% or less, and the modified PVA resin containing such an amount of the unsaturated monomer units may have improved solubility in water.

The degree of saponification of the modified PVA resin is preferably, but not limited to, 80 mol% or more (e.g., 80 to 99.9 mol%), more preferably 88 mol% or more (e.g., 88 to 99.9 mol%), and further preferably 95 mol% or more (e.g., 95 to 99.9 mol%) .

The viscosity of the modified PVA resin may vary. A 4 mass% aqueous solution of the modified PVA resin (at 20°C) has a viscosity of preferably 2 to 500 mPa·s, more preferably 3 to 300 mPa·s, and further preferably 5 to 200 mPa·s (e.g., 5 to 80 mPa·s).

The degree of saponification and the viscosity of a 4 mass% aqueous solution may be measured in accordance with JIS K 6726.

Polyvinyl alcohol resin having triad syndiotacticity of 32 to 40% (A2)

The polyvinyl alcohol resin (A) may preferably be a polyvinyl alcohol resin having a triad syndiotacticity of 32 to 40% (A2) (simply also called the highly syndiotactic PVA resin herein).

The highly syndiotactic PVA resin preferably has a triad syndiotacticity of 32 to 40%, more preferably 33 to 39%, and further preferably 34 to 38%. The highly syndiotactic PVA resin having a triad syndiotacticity of 32% or more easily forms an aqueous gel, and the highly syndiotactic PVA resin having a triad syndiotacticity of 40% or less can easily form an aqueous gel.

The triad syndiotacticity can be determined from the peaks attributed to hydroxy groups in the proton NMR analysis of the highly syndiotactic PVA resin dissolved in deuterated dimethylsulfoxide (DMSO).

The highly syndiotactic PVA resin may be produced by any method that yields the highly syndiotactic PVA resin having a triad syndiotacticity of 32 to 40%. The highly syndiotactic PVA resin can easily be produced by saponifying a vinyl ester polymer prepared by a conventionally known method.

That is, the highly syndiotactic PVA resin is a saponified product of a vinyl ester polymer.

The vinyl ester polymer may be produced by any method that includes polymerization of vinyl ester monomers, and may be performed in accordance with a conventionally known method.

The shape of a polymerization vessel, the type of a polymerization agitator, the polymerization temperature, the pressure in the polymerization vessel, or other conditions may also be in accordance with a conventionally known method. The polymerization method may be selected from various conventionally known methods, including bulk polymerization, solution polymerization, suspension polymerization, emulsion polymerization, or other polymerization methods. For ease of controlling the degree of polymerization or performing a saponification reaction after polymerization, preferred polymerization methods are, but not limited to, solution polymerization using an alcohol as a solvent, or suspension polymerization using water or a combination of water and an alcohol as a dispersion medium.

Examples of the vinyl ester monomers include vinyl esters, such as fatty acid vinyl esters and non-fatty acid vinyl esters, e.g., vinyl formate, aromatic carboxylic acid vinyl esters, etc. For production of a PVA having a high syndiotacticity, the vinyl ester monomers are $C_{3-15}$ fatty acid vinyl esters, e.g., linear or branched $C_{3-15}$ fatty acid vinyl esters, such as vinyl propionate, vinyl butyrate, and vinyl pivalate, and are preferably $C_{3-10}$ fatty acid vinyl esters, such as linear or branched $C_{3-10}$ fatty acid vinyl esters; $C_{3-15}$ fatty acid vinyl esters having one or more substituents (e.g., a halogen group), such as vinyl trifluoroacetate and vinyl trichloroacetate; vinyl formate; etc. These vinyl esters can be used alone or in combination of two or more.

Specific examples of the method for producing the highly syndiotactic PVA include a method including homopolymerization or copolymerization of vinyl ester monomers having a bulky side chain, such as vinyl propionate, vinyl butyrate, and vinyl pivalate, followed by saponification of the product using an alkaline catalyst; and a method including homopolymerization or copolymerization of vinyl ester monomers having a high polarity, such as vinyl formate, vinyl trifluoroacetate, and vinyl trichloroacetate, followed by saponification of the product using an alkaline catalyst. Preferred method includes polymerization of vinyl pivalate followed by saponification of the product using an alkaline catalyst. An example of a production process of a PVA resin having a triad syndiotacticity of 37.1% will be described in Examples later. The triad syndiotacticity of the PVA resin can be reduced to less than 37.1% by, for example, adding vinyl acetate to the polymerization reaction of vinyl pivalate to give a vinyl pivalate-vinyl acetate copolymer, or by raising the polymerization temperature. The triad syndiotacticity of the PVA resin can be increased to more than 37.1% by, for example, lowering the polymerization temperature in the example of the production process. In any case, the triad syndiotacticity of the resulting highly syndiotactic PVA can be determined from the peaks attributed to hydroxy groups in the proton NMR analysis of the highly syndiotactic PVA resin dissolved in deuterated DMSO. The highly syndiotactic PVA resin determined to have a triad syndiotacticity of 32 to 40% can be selected as appropriate and used in the present invention.

The vinyl ester polymer containing the above vinyl ester monomers may further contain an additional unsaturated monomer capable of copolymerizing with the vinyl ester monomers to the extent that the additional unsaturated monomer does not impair the effects of the invention.

The additional unsaturated monomer may be one or more unsaturated monomers selected from carboxyl group-containing unsaturated monomers, such as (meth) acrylic acid, maleic acid, maleic anhydride, fumaric acid, crotonic acid, itaconic acid, and undecylenic acid; unsaturated dibasic acid monoalkyl esters, such as monomethyl maleate and monomethyl itaconate; amide group-containing unsaturated monomers, such as acrylamide, dimethylacrylamide, dimethylaminoethylacrylamide, diethylacrylamide, dimethylaminopropylacrylamide, isopropylacrylamide, N-methylolacrylamide, and N-vinylacetamide; vinyl halides, such as vinyl chloride and vinyl fluoride; glycidyl group-containing unsaturated monomers, such as allyl glycidyl ether and glycidyl methacrylate; lactam group-containing unsaturated monomers including, e.g., N-vinylpyrrolidones such as N-vinyl-2-pyrrolidone and N-vinyl-alkylpyrrolidone (e.g., N-vinyl-mono- or di-$C_{1-4}$ alkyl-pyrrolidones, such as N-vinyl-3-propyl-2-pyrrolidone, N-vinyl-5-methyl-2-pyrrolidone, N-vinyl-5-ethyl-2-pyrrolidone, N-vinyl-5,5-dimethyl-2-pyrrolidone, and N-vinyl-3,5-dimethyl-2-pyrrolidone), N-allylpyrrolidones such as N-allyl-2-pyrrolidone, N-vinylpiperidones such as N-vinyl-2-piperidone and N-vinyl-alkylpiperidone (e.g., N-vinyl-

mono- or di-$C_{1-4}$ alkyl-piperidones, such as N-vinyl-6-methyl-2-piperidone and N-vinyl-6-ethyl-2-piperidone), and N-vinylcaprolactams such as N-vinyl-ε-caprolactam and N-vinyl-alkylcaprolactam (e.g., N-vinyl-mono- or di-$C_{1-4}$ alkyl-caprolactams, such as N-vinyl-7-methyl-2-caprolactam and N-vinyl-7-ethyl-2-caprolactam); alkyl vinyl ethers such as $C_{1-20}$ alkyl vinyl ethers (e.g., methyl vinyl ether, n-propyl vinyl ether, i-propyl vinyl ether, n-butyl vinyl ether, i-butyl vinyl ether, t-butyl vinyl ether, lauryl vinyl ether, dodecyl vinyl ether, and stearyl vinyl ether); nitriles such as acrylonitrile and methacrylonitrile; hydroxy group-containing unsaturated monomers, including, e.g., $C_{1-20}$ monoalkyl allyl alcohols such as allyl alcohol and isopropenyl allyl alcohol, $C_{1-20}$ dialkyl allyl alcohols such as dimethyl allyl alcohol, and hydroxy $C_{1-20}$ alkyl vinyl ethers such as hydroxy ethyl vinyl ether and hydroxy butyl vinyl ether; acetyl group-containing unsaturated monomers, including, e.g., $C_{1-20}$ alkyl allyl acetates such as allyl acetate, dimethylallyl acetate, and isopropenylallyl acetate; (meth)acrylic acid esters, including, e.g., (meth)acrylic acid alkyl esters, such as (meth) acrylic acid $C_{1-20}$ alkyl esters such as methyl (meth)acrylate, ethyl (meth)acrylate, 2-ethylhexyl acrylate, and n-butyl acrylate; vinylsilanes such as trimethoxyvinylsilane, tributylvinylsilane, and diphenylmethylvinylsilane; polyoxyalkylene (meth)acrylates, such as polyoxyethylene (meth)acrylate and polyoxypropylene (meth)acrylate; polyoxyalkylene (meth)acrylamides, such as polyoxyethylene (meth)acrylamide and polyoxypropylene (meth)acrylamide; polyoxyalkylene vinyl ethers, such as polyoxyethylene vinyl ether and polyoxypropylene vinyl ether; polyoxyalkylene alkylvinyl ethers, such as polyoxyethylene allyl ether, polyoxypropylene allyl ether, polyoxyethylene butylvinyl ether, and polyoxypropylene butylvinyl ether; α-olefins such as ethylene, propylene, n-butene, and 1-hexene; butenes such as 3,4-dihydroxy-1-butene, 3,4-diacyloxy-1-butene, 3-acyloxy-4-hydroxy-1-butene, 4-acyloxy-3-hydroxy-1-butene, and 3,4-diacyloxy-2-methyl-1-butene; pentenes such as 4,5-dihydroxy-1-pentene, 4,5-diacyloxy-1-pentene, 4,5-dihydroxy-3-methyl-1-pentene, and 4,5-diacyloxy-3-methyl-1-pentene; hexenes such as 5,6-dihydroxy-1-hexene and 5,6-diacyloxy-1-hexene; unsaturated amine monomers, such as N,N-dimethylallylamine, N-allylpiperazine, 3-piperidine acrylic acid ethyl ester, 2-vinylpyridine, 4-vinylpyridine, 2-methyl-6-vinylpyridine, 5-ethyl-2-vinylpyridine, 5-butenylpyridine, 4-pentenylpyridine, and 2-(4-pyridyl)allyl alcohol; quaternary ammonium compound-containing unsaturated monomers, such as dimethylaminoethyl acrylate methyl chloride quaternary salt, N,N-dimethylaminopropyl acrylamide methyl chloride quaternary salt, and N,N-dimethylaminopropyl acrylamide methyl benzenesulfonate quaternary salt; aromatic unsaturated monomers such as styrene; sulfonic acid group-containing unsaturated monomers, such as 2-acrylamide-2-methylpropanesulfonic acid or its alkali metal salts, ammonium salts or organic amine salts, 2-acrylamide-1-methylpropanesulfonic acid or its alkali metal salts, ammonium salts or organic amine salts, 2-methacrylamide-2-methylpropanesulfonic acid or its alkali metal salts, ammonium salts or organic amine salts, vinyl sulfonic acid or its alkali metal salts, ammonium salts or organic amine salts, allyl sulfonic acid or its alkali metal salts, ammonium salts or organic amine salts, and methallyl sulfonic acid or its alkali metal salts, ammonium salts or organic amine salts; glycerol monoallyl ether; 2,3-diacetoxy-1-allyloxypropane; 2-acetoxy-1-allyloxy-3-hydroxypropane; 3-acetoxy-1-allyloxy-3-hydroxypropane; 3-acetoxy-1-allyloxy-2-hydroxypropane; glycerol monovinyl ether; glycerol monoisopropenyl ether; acryloyl morpholine; vinyl ethylene carbonate; vinylimidazole; and vinylcarbazole.

The amount of the additional unsaturated monomer may be, for example, but is not limited to, 10 mol or less relative to 100 mol of the vinyl ester monomers.

A polymerization catalyst may be used for the polymerization. The polymerization catalyst may typically be, but is not limited to, an azo compound or a peroxide.

An organic acid, such as tartaric acid, citric acid, and acetic acid, may be added to the polymerization reaction to prevent the hydrolysis of the fatty acid vinyl ester.

A polymerization terminator may be used to terminate the polymerization. The polymerization terminator may be, for example, but is not limited to, m-dinitrobenzene etc.

The polymerization temperature may be set at any temperature known to be used in polymerization. For ease of production of a PVA resin having a high syndiotacticity and other reasons, the polymerization temperature is, for example, -50 to 200°C, preferably -50 to 150°C, and more preferably 0 to 120°C.

The vinyl ester polymer is produced in the manner as described above. The resulting polymer may be saponified by any conventionally known method. For example, a conventionally known alcoholysis or hydrolysis is applicable using a basic catalyst, such as sodium hydroxide, potassium hydroxide, and sodium methoxide, or an acidic catalyst, such as hydrochloric acid, sulfuric acid, and p-toluenesulfonic acid. The syndiotacticity of the polymer is usually not changed before and after the saponification reaction.

The solvent used in the saponification may be, for example, an alcohol such as methanol and ethanol; an ester such as methyl acetate and ethyl acetate; a ketone such as acetone and methyl ethyl ketone; an aromatic hydrocarbon such as benzene and toluene; tetrahydrofuran; etc. These solvents may be used alone or in combination of two or more. The temperature, duration, and other conditions of the saponification are not particularly limited.

The drying, grinding or washing of the saponified product may be done by any methods and may be performed by a conventionally known method.

A saponified product of the vinyl ester polymer, i.e., the highly syndiotactic PVA resin according to the present invention is produced in the manner as described above. The produced highly syndiotactic PVA resin may be post-modified by acetalization, urethanation, etherification, grafting, phosphorylation, acetoacetylation, cationization, or other reactions following a known method to the extent that the effects of the present invention are not impaired.

The degree of saponification of the highly syndiotactic PVA resin is preferably 90 to 99.9 mol%, more preferably 98 to 99.9 mol%, and further preferably 99 to 99.9 mol%. The degree of saponification of the highly syndiotactic PVA resin can be determined by, for example, proton NMR analysis in deuterated DMSO solution.

The degree of polymerization of the highly syndiotactic PVA resin is preferably 100 to 10, 000, more preferably 500 to 8, 000, and further preferably 1,000 to 5, 000. For ease of handling, the degree of polymerization is particular preferably 1, 000 to 3, 000. The highly syndiotactic PVA resin having a degree of polymerization of 100 or more can be used to easily produce an aqueous gel having a high resin strength (stress) and shape retainability. When the degree of polymerization is 10, 000 or less, an aqueous solution of the resin can easily be handled. The degree of polymerization is measured before saponification and is determined in terms of the degree of polymerization of polyvinyl acetate in a benzene solution at 30°C in accordance with JIS K 6725.

Polyvinyl alcohol resin having degree of saponification of 97 mol% or more (A3)

The polyvinyl alcohol resin (A) may be a polyvinyl alcohol resin having a degree of saponification of 97 mol% or more (A3) (simply also called the completely saponified PVA resin herein) as described above.

The degree of saponification of the completely saponified PVA resin is preferably 97 mol% or more (e.g., 97 to 99.9 mol%), more preferably 98 mol% or more (e.g., 98 to 99.9 mol%), and particularly preferably 98.5 mol% or more (e.g., 98.5 to 99.9 mol%) .

The degree of polymerization of the completely saponified PVA resin is, for example, 100 to 10,000, more preferably 500 to 9, 000, further preferably 1, 000 to 8, 000, and particularly preferably 1,500 to 5, 000.

Crosslinking agent

The angiogenesis device may further comprise a crosslinking agent.

The crosslinking agent may be any crosslinking agent and may be selected depending on the type of polymer or other factors . For example, when the modified PVA resin (A1) is used as the polymer to produce the angiogenesis device, a suitable crosslinking agent may be a crosslinking agent having a functional group (e.g., a hydrazino group etc.) capable of reacting with the carbonyl groups of the modified PVA resin (A1).

Examples of the crosslinking agent include hydrazide compounds, semicarbazide compounds, or the like. Particularly preferred are hydrazide compounds, semicarbazide compounds, or the like having two or more functional groups selected from the groups represented by the formulas (1) to (3) below in the molecule. These compounds can be used alone or in combination of two or more.

$$-NH-NH_2 \qquad (1)$$

$$-CO-NH-NH_2 \qquad (2)$$

$$-NH-CO-NH-NH_2 \qquad (3)$$

Specific examples of the hydrazide compounds include carbohydrazide; dicarboxylic acid hydrazides, including aliphatic dicarboxylic acid hydrazides, such as oxalic acid dihydrazide, malonic acid dihydrazide, succinic acid dihydrazide, glutaric acid dihydrazide, adipic acid dihydrazide, pimelic acid dihydrazide, suberic acid dihydrazide, azelaic acid dihydrazide, sebacic acid dihydrazide, dodecanedioic acid dihydrazide, and hexadecanedioic acid dihydrazide; aromatic dicarboxylic acid hydrazides, such as terephthalic acid dihydrazide, isophthalic acid dihydrazide, 2,6-naphthoic acid dihydrazide, and 4,4'-bisbenzene dihydrazide; alicyclic dicarboxylic acid hydrazides, such as 1,4-cyclohexanedicarboxylic acid dihydrazide; hydroxyl group-containing dicarboxylic dihydrazides, such as tartaric acid dihydrazide and malic acid dihydrazide; iminodiacetic acid dihydrazide; itaconic acid dihydrazide; 1,3-bis(hydrazinocarbonoethyl)-5-isopropylhydantoin; 7,11-octadecadiene-1,18-dicarbohydrazide; tris(2-hydrazinocarbonylethyl)isocyanurate; citric acid trihydrazide; butanetricarbohydrazide; 1,2,3-benzenetrihydrazide; ethylenediaminetetraacetic acid tetrahydrazide; 1,4,5,8-naphthoic acid tetrahydrazide; nitriloacetic acid trihydrazide; cyclohexanetricarboxylic acid trihydrazide; pyromellitic acid tetrahydrazide; etc.

Examples of the semicarbazide compounds include N,N'-hexamethylene bissemicarbazide, and biuretrytri(hexamethylenesemicarbazide) .

Derivatives produced by a reaction of these hydrazide compounds or semicarbazide compounds with low boiling point ketones, such as acetone and methyl ethyl ketone, may also be used.

Dicarboxylic acid hydrazides, polyacrylic acid hydrazide and the like are preferred, adipic acid dihydrazide, polyacrylic acid hydrazide and the like are more preferred, and polyacrylic acid hydrazide is particularly preferred among the crosslinking agents as exemplified above. These crosslinking agents are suitable for the invention due to low toxicity, high solubility in water, and other advantages.

The crosslinking agents as described above may be used alone or in combination of two or more.

The amount of the crosslinking agent(s) contained in the polymer is preferably 1 to 30 parts by mass, more preferably 2 to 25 parts by mass, and further preferably 3 to 20 parts by mass (e.g., 4 to 15 parts by mass) relative to 100 parts by mass of the polymer (e.g., the modified PVA resin).

The amount of the crosslinking agent (s) is 1 part by mass or more such that high crosslinking density and sufficient strength (stress) as the angiogenesis device are achieved. The amount of the crosslinking agent (s) is 30 parts by mass or less such that residual unreacted crosslinking agent(s) can be minimized.

When the crosslinking agent is polyacrylic acid hydrazide, the weight average molecular weight (Mw) of the polyacrylic acid hydrazide is preferably, but not limited to, about 3, 000 to 6,000,000, more preferably about 5,000 to 1,000,000, and further preferably about 8, 000 to 800, 000 (e.g., about 10,000 to 300, 000, about 1, 000 to 200, 000, or about 10, 000 to 100, 000).

When the crosslinking agent is polyacrylic acid hydrazide, the degree of hydrazidation of the polyacrylic acid hydrazide is preferably, but not limited to, 30% or more, more preferably 50% or more, further preferably 70% or more, and particularly preferably 80% or more.

The molecular weight and the degree of hydrazidation of polyacrylic acid hydrazide may be adjusted as appropriate to the extent that the effects of the present invention are not impaired. For example, when the molecular weight of polyacrylic acid hydrazide is small, the degree of hydrazidation is adjusted to be large. When the molecular weight of polyacrylic acid hydrazide is large, the degree of hydrazidation is adjusted to be small.

Cell culture components

The angiogenesis device may further contain a cell culture component.

The cell culture component may be, for example, but is not limited to, an alkali metal, an alkaline earth metal, a halogen, glucose, etc. Suitable cell culture component is an acetate buffer solution or a phosphate buffer solution containing Na, K, Cl, Ca, glucose and/or others.

When the cell culture component contains Na, the concentration of Na may be adjusted to preferably 20 to 150 mEq/L, more preferably 80 to 140 mEq/L.

When the cell culture component contains K, the concentration of K may be adjusted to preferably 2.5 to 130 mEq/L, more preferably 3.5 to 40 mEq/L.

When the cell culture component contains Cl, the concentration of Cl may be adjusted to preferably 15 to 170 mEq/L, more preferably 100 to 150 mEq/L.

When the cell culture component contains Ca, the concentration of Ca may be adjusted to preferably 0.5 to 5 mEq/L, more preferably 1 to 3 mEq/L.

When the cell culture component contains glucose, the concentration of glucose may be adjusted to preferably 1 to 11 mM, more preferably 3 to 7 mM.

The cell culture component may also be, for example, a known cell culture medium, such as Hanks' balanced salt solution (HBSS); a commercially available preservation solution, such as Euro-Collins solution, CELLBANKER, and UW solution (University of Wisconsin solution) ; a cell protective component, such as dimethylsulfoxide (DMSO) and serum albumin; a component for preventing the contamination of germs, such as antibiotics; a component for maintaining the activity of cells, such as vitamins such as nicotinamide; etc. The cell culture component is preferably a known cell culture medium etc.

These cell culture components can be used alone or in combination of two or more.

Such a cell culture component may be used in combination with another component, such as a sustained release agent, an isotonic agent, and a pH adjusting agent.

The angiogenesis device may further contain a component other than those described above. The angiogenesis device may further contain, for example, a cell growth factor, which promotes or controls the growth of live cells; a cytokine, which is an active substance produced from cells; other physiologically active substances; a blood-flow accelerator that promotes the blood flow to the angiogenesis device; a neurotrophic factor; etc.

These cell culture components can be used alone or in combination of two or more.

Examples of the cell growth factor include epidermal growth factor (EGF), hepatocyte growth factor (HGF) and insulin.

Examples of the cytokine include hematopoietic factors, such as interleukins, chemokines and colony-stimulating factors, tumor necrosis factors, and interferons.

Examples of other physiologically active substances include amino acids, such as glycine, phenylalanine, lysine, aspartic acid, and glutamic acid; vitamins, such as biotin, pantothenic acid, and vitamin D; serum albumin; and antibiotics.

Examples of the blood-flow accelerator include citrulline or salts thereof, capsaicin, and capsaicinoids.

Examples of the neurotrophic factor include nerve growth factor (NGF), brain-derived neurotrophic factor (BDNF), neurotrophin-3 (NT-3), neurotrophin-4 (NT-4), glial cell-derived neurotrophic factor (GDNF), neurturin, artemin, and persephin.

The amount of these components added to the angiogenesis device is not limited to a particular amount.

Gel

The polymer contained in the angiogenesis device, or the polymer containing a non-polymer component (e.g., the angiogenic component, the cell culture component, etc.) may be in the form of a gel, in particular, an aqueous gel (hydrogel) . The formation of such a gel may be performed as appropriate for the type of polymer. For example, the gel may be formed by crosslinking the polymer with a crosslinking agent described later or other agents (gel crosslinking), or a polymer capable of forming a gel (e.g., the polyvinyl alcohol resin (A2) as described above) may be used to form a gel.

The concentration of the polymer in the gel may be, for example, 0.3 to 20%, and is preferably 0.5 to 10%, more preferably 1 to 8%, for example, 3 to 8%. The gel containing such a percentage of the polymer is preferred because the gel is capable of maintaining the shape of the angiogenesis device implanted in the body of an animal for a long period of time and has other advantages.

When a crosslinking agent is contained in the gel or angiogenesis device, the amount of the crosslinking agent can be selected as appropriate for the type of crosslinking agent, the desired strength of the gel or other factors. The amount of the crosslinking agent may be, for example, 0.5 parts by mass or more, and is preferably 1 part by mass or more, more preferably 3 parts by mass or more, etc. relative to 100 parts by mass of the polymer. The amount of the crosslinking agent may be, for example, 20 parts by mass or less, and is preferably 18 parts by mass or less, more preferably 15 parts by mass or less, etc. relative to 100 parts by mass of the polymer.

When the gel containing the angiogenic component (e.g., MSCs) is prepared, the percentage of viable cells in the gel or the angiogenesis device relative to the total live cells in the angiogenic component (e.g., MSCs) before added to the gel or the angiogenesis device may be, for example, 60 to 100%, and is preferably 70 to 100%, more preferably 80 to 100%.

The number of viable cells can be measured by, for example, cytoplasm staining using fluorescein diacetate reagent (may be abbreviated to FDA measurement), or nucleus staining using propidium iodide (may be abbreviated to PI measurement).

The gel typically has a predetermined strength (stress). The gel may sustain a sufficient stress not to easily break when the angiogenesis device is implanted or retrieved. The strength of the gel may vary depending on the type of polymer (e.g., the type of polyvinyl alcohol resin, the viscosity of a 4% aqueous solution of the resin, the degree of saponification, the degree of modification, syndiotacticity, etc.), the type of crosslinking agent, the amount of the crosslinking agent added, and the solid content of the gel. The strength of the gel is therefore not limited to a specific value, but the gel may be able to sustain a stress of, for example, about 0.1 kPa or more, e.g., about 0.3 kPa or more, or may be 0.4 kPa or more, e.g., 0.5 kPa or more, e.g., 0.5 to 100 kPa, and is preferably 0.55 kPa or more, e.g., 0.6 kPa or more, e.g., 0.6 to 95 kPa, more preferably 0.65 kPa or more, e.g., 0.7 to 90 kPa, and further preferably 0.7 kPa or more, e.g., 0.7 to 85 kPa, as measured at 20°C.

The upper limit of the stress (at 20°C) is not limited, but is preferably not excessively large so that the vascular bed or the surrounding tissue around the angiogenesis device is prevented from being damaged when the angiogenesis device is implanted, placed or retrieved. The upper limit of the stress sustained by the gel may be, for example, 100 kPa, 95 kPa, 90 kPa, 85 kPa, 80 kPa, 75 kPa, 70 kPa, 65 kPa, 60 kPa, 55 kPa, 50 kPa, 45 kPa, 40 kPa, 35 kPa, 30 kPa, 25 kPa, 20 kPa, 15 kPa, 12 kPa, 10 kPa, 9 kPa, 8 kPa, etc.

Such a stress value (at 20°C) may be satisfied or sustained by the gel to be implanted or placed, or such a stress may be satisfied or sustained by the gel when the angiogenesis device is implanted or placed, or when the angiogenesis device is retrieved after implantation or placement for a predetermined period of time.

The stress sustained by the gel may be measured using, for example, a compact tabletop-tester EZ Test EZ-SX (Shimadzu Corporation) following the manufacturer's instructions. In particular, the stress may be measured by the method described in Examples later.

The gel, in particular the aqueous gel, may be in any shape, and may be in the shape of, for example, not limited to, a sheet, a plate, a disk, a bar, a tube, a bead, etc.

The size of the gel, in particular the aqueous gel, may be selected as appropriate for a site for implantation of the angiogenesis device (angiogenesis site) and its size, etc., and is not particularly limited. For example, the thickness of the gel is preferably 0.1 to 10 mm, more preferably 0.2 to 5 mm, further preferably 0.5 to 2 mm, and particularly preferably 0.7 to 1.5 mm.

<u>Configuration etc.</u>

The angiogenesis device comprises the angiogenic component and the polymer as described above, and optionally an additional component. These components may be in any configuration, but typically the angiogenic component may be present within the polymer, i.e., inside of the angiogenesis device.

Such a configuration may be realized, for example, as a configuration in which the angiogenic component is present within the polymer, e.g., dissolved or dispersed in the polymer, or as a configuration in which the polymer surrounds the angiogenic component. The angiogenic component is preferably present within the polymer, and the angiogenic component is more preferably present within a gel formed of the polymer.

The dissolved or dispersed state of the angiogenic component can be examined by, for example, visual observation under a microscope, or luminescence imaging system using an angiogenic component (e.g., MSCs) derived from luciferase transgenic s, or other techniques. Luminescence imaging system is an in vivo semi-quantitative assay system using a highly sensitive cooled camera to detect very weak luminescence or fluorescence that is invisible to the naked eye. An angiogenic component (e.g., animal-derived MSCs) carrying the firefly luciferase gene that emits light at a wavelength of 600 nm or more may be implanted into a healthy animal, and luciferin serving as a substrate for luciferase is administered to the animal to cause a luminescence reaction to occur, thereby allowing observation of in vivo biological response in the animal over time.

The configuration as described above facilitates relatively uniform distribution of the angiogenic component, or easily prevents aggregation of the angiogenic component. Due to these advantages, oxygen and nutrients may be efficiently supplied to the cells, and may efficiently promote angiogenesis, i.e., may allow the angiogenic component to exhibit sufficient functions without reduction of its viability.

The angiogenesis device may contain a scaffold or may have a scaffold function. In particular, the angiogenic component may be contained in a scaffold. Such a scaffold is capable of securing the angiogenic component (e.g., MSCs) therein to an implantation site and serving as a supporting framework that allows the angiogenic component (e.g., MSCs) to exhibit its functions at the implantation site.

The scaffold capable of sufficiently securing the angiogenic component (e.g., MSCs) to an implantation site prevents or inhibits the diffusion of the angiogenic component (e.g., MSCs) and promotes induction of efficient angiogenesis at a target site.

The scaffold capable of exhibiting a sufficient scaffold function (i.e., a function as a supporting framework) allows the angiogenic component to efficiently exhibit its angiogenesis-promoting function. For example, when the angiogenic component is, for example, MSCs, a growth factor or other factors required for angiogenesis are efficiently secreted.

The securing function of the scaffold can be examined by, for example, the luminescence imaging system as described above or other techniques.

The scaffold function (i.e., a function as a supporting framework) of the scaffold can be examined by, for example, immunostaining vWF (von Willebrand factor) at the site in which the angiogenesis device has been implanted. In particular, the subcutaneous tissue around the angiogenesis device is harvested, fixed in 4% paraformaldehyde overnight, embedded in paraffin, and sectioned into thin sections. The specimen is reacted with anti-vWF antibody (AB7356; Merck Millipore) as a primary antibody for 30 minutes, and then reacted with EnVision System-HRP (Dako) as a secondary antibody for 30 minutes. Color development is performed using diaminobenzidine (DAB) (DAKO), and the number of positive cells is counted.

The scaffold preferably rarely forms adhesions to the surrounding tissue, i.e., preferably has an adhesion preventive ability. The adhesion preventive ability refers to, for example, the capability of preventing or inhibiting formation of adhesions between the device and the surrounding tissue when the device is placed at an implantation site.

The scaffold having an adhesion preventive ability facilitates retrieval of the angiogenesis device after formation of new blood vessels, and easily prevents bleeding, inflammation or release of exudates caused by injury, damage or rupture of the surrounding tissue or newly formed blood vessels.

The scaffold may typically be made of a polymer, in particular, the polymer as described above. In particular, the material of the scaffold may be selected from, but is not limited to, structural proteins such as collagen, elastin and keratin; gelatin; glycosaminoglycans such as hyaluronic acid, chondroitin sulfate, keratan sulfate, dermatan sulfate, heparan sulfate and heparin; proteoglycans; cell adhesion molecules such as fibronectin, laminin, fibrinogen and vitronectin; fibrin; fibroin; sericin; alginic acid; chitosan; agarose; cellulose; cellulose derivatives such as cellulose nanofibers, carboxymethyl cellulose, and hydroxypropyl cellulose; synthetic polypeptides; polylactic acid; polyglycolic acid; polycaprolactone; polyethylene glycol; polypropylene glycol; 2-methacryloyloxyethyl phosphorylcholine; poly(meth)acrylic acid; polyacrylamide; poly-N-isopropylacrylamide; polyvinyl alcohol resins; etc.

These materials (polymers) may be used alone or in combination of two or more to form the scaffold.

A polyvinyl alcohol resin, in particular, at least one of the polyvinyl alcohol resins (A1) to (A3), in particular, at least one of the polyvinyl alcohol resins (A1) and (A2) may be used to form the scaffold. A gel made from such a polyvinyl alcohol resin may be used to form the scaffold.

Such a polyvinyl alcohol resin efficiently exhibits an excellent scaffold function and an excellent adhesion preventive ability as described above, and is thus suitable to form the scaffold.

An adhesion preventive layer, i.e., a layer with an adhesion preventive ability may further be provided on the outer periphery or exterior of the scaffold or as an outer layer on the scaffold. The adhesion preventive layer may be made of a polymer, in particular, the polymer as described above. In particular, the material of the adhesion preventive layer may be selected from, for example, but is not limited to, dextrin; hyaluronic acid; alginic acid; cellulose; cellulose derivatives such as cellulose nanofibers, carboxymethyl cellulose, and hydroxypropyl cellulose; silicone; polyurethane; fluororesins such as polytetrafluoroethylene and perfluoroalkoxyalkane; polyvinyl alcohol resins; and others.

These materials may be used alone or in combination of two or more to form the adhesion preventive layer.

A polyvinyl alcohol resin, in particular, at least one of the polyvinyl alcohol resins (A1) to (A3), in particular, at least one of the polyvinyl alcohol resins (A1) and (A2) may be used to form the adhesion preventive layer. A gel made from such a polyvinyl alcohol resin may be used to form the adhesion preventive layer.

Such a polyvinyl alcohol resin efficiently exhibits an excellent adhesion preventive ability as described above, and is thus suitable to form the adhesion preventive layer.

The adhesion preventive layer may be integrated with the scaffold. The adhesion preventive layer may be made of the same or different polymer used to form the scaffold. In particular, the adhesion preventive layer may be made of a polymer that is a similar type to the polymer used to form the scaffold or may be made of a gel formed from such a type of polymer. The adhesion preventive layer is preferably made of the same polymer as that used to form the scaffold or made of a gel formed from the polymer.

The angiogenic component is typically present within the polymer, i.e., within or in the polymer that is inside of the angiogenesis device, as described above.

Such a configuration of the angiogenic component in relation to the scaffold (or a scaffold function) and the adhesion preventive layer (or an adhesion preventive function) may be, for example, a configuration in which the angiogenic component is contained in the scaffold made of a polymer and/or the adhesion preventive layer, wherein the scaffold may be a polymer having a scaffold function and optionally an adhesion preventive function, especially, a gel formed from such a polymer, and wherein the adhesion preventive layer may be a polymer having an adhesion preventive function, especially, a gel formed from such a polymer.

A typical angiogenesis device typically contains at least the angiogenic component in the scaffold made of a polymer (especially, a gel formed from a polymer). When such an angiogenesis device further contains the adhesion preventive layer, the adhesion preventive layer may contain the angiogenic component or may not contain the angiogenic component.

The angiogenesis device may further contain a supporting substrate.

In particular, the gel, for example, the gel that forms the scaffold, may contain a supporting substrate useful as a reinforcement for the purpose of reinforcing the gel and/or for ease of handling.

For example, when the gel (in particular, in the form of an aqueous gel) is formed into a thin film, a polymer is placed on a substrate (a reinforcement), such as a mesh sheet made of a resin, for reinforcement and/or for ease of handling, and is then formed into a gel.

The material of the supporting substrate may be, but is not limited to, a polymer such as PET (polyethylene terephthalate), PE (polyethylene), PP (polypropylene), and Teflon (registered trademark); a metal; etc. The material of the supporting substrate preferably does not degrade or deteriorate in a living body, but may be a biodegradable material that degrades in a living body after a certain period of time has passed.

The mesh size of the mesh sheet is such a size that allows permeation of molecules that should be permeated and have an assumed maximum diameter of about 5 nm, such as oxygen, inorganic or organic nutrients and various hormones (e.g., physiologically active substances including hormones, such as insulin) while preventing permeation of molecules that should be prevented from permeating and have an assumed minimum diameter of about 50 nm, such as immune-related cells and immune-related substances (e.g., antibodies, complements, etc.). The mesh size of the mesh sheet is typically 5 to 100 nm, preferably 10 to 50 nm, and more preferably 20 to 30 nm.

The amount of the angiogenic component contained in the angiogenesis device in terms of, e.g., the number of cells is preferably, for example, but not limited to, $1 \times 10°$ to $1 \times 10^5$ cells/mm$^2$, more preferably $5 \times 10°$ to $5 \times 10^4$ cells/mm$^2$, and particularly preferably $1 \times 10^1$ to $1 \times 10^4$ cells/mm$^2$.

When the angiogenic component is contained in such an amount, both of a sufficient angiogenesis function and a preventive or inhibitory effect on bleeding, inflammation and/or release of exudates may be much more efficiently achieved.

The angiogenesis device or the gel contained in the angiogenesis device preferably slightly shrink during the placement in the body. The rate of volume shrinkage or the rate of volume change before and after the placement in the body for one month is preferably, but not limited to, 10% or more, e.g., 20% or more, e.g., 20 to 90%, more preferably 25% or more, e.g., 30% or more, e.g., 30 to 80%, and particularly preferably 35% or more, e.g., 40% or more, e.g., 40 to 70%.

The temperature of a site for placement of the angiogenesis device may be a predetermined temperature, e.g., 37°C.

In the determination of the rate of volume change, the volume change during the placement in the body can be simulated as the volume change during the placement in water, i.e., the volume change before and after immersion in water.

In other words, in the determination of the rate of volume change, the placement in the body for one month is simulated as the placement or immersion in water for one month.

The rate of volume change can be calculated by the following formula:

$$\text{Rate of volume change (\%)} = \text{volume after placement or immersion in water for}$$

$$\text{one month/volume before placement or immersion in water} \times 100$$

The angiogenesis device or gel having such a rate of shrinkage is easily detached from the surrounding tissue, which makes easy to retrieve the device from the body. Such an angiogenesis device or gel may reduce damage to the angiogenic component (e.g., MSCs), and may easily create an implantation pocket where a cell-embedding device is to be implanted after retrieval of the angiogenesis device.

By adjusting the rate of shrinkage (the rate of volume change) and the stress, ease of placement and retrieval of the angiogenesis device can be efficiently achieved or may be in good balance.

The rate of shrinkage can be adjusted as appropriate depending on the selected factors, including the material and physical properties of the device (the type of polymer, the strength of the gel, etc.) and the duration of placement.

Production methods

The angiogenesis device can be produced by combining an angiogenic component and a polymer, and optionally an additional component.

The angiogenesis device can be produced by a conventional method selected as appropriate to the types, forms, configurations, etc. of the components as in the manner specifically described below.

For example, a liquid mixture containing a polymer and optionally an additional component is preferably formed into a gel.

The polymer is formed or shaped into a gel by, for example, pouring a liquid mixture (in particular, an aqueous solution or optionally in a sol form) containing a polymer containing, e.g., a polyvinyl alcohol resin and optionally an angiogenic component (e.g., MSCs), a crosslinking agent and a cell culture component as needed into a mold having a desired shape to allow gelation of the mixture; or, alternatively, by cutting a gel prepared from such a mixture into a desired shape with a knife etc.

The liquid mixture (in particular, an aqueous solution) containing a polymer containing, e.g., a polyvinyl alcohol resin and optionally an angiogenic component (e.g., MSCs), a crosslinking agent and a cell culture component as needed undergoes a sol state to become a gel. Such a sol is understood to be an equivalent of the gel according to the present invention and is within the scope of the present invention.

The solid content of the liquid mixture (in particular, an aqueous solution or optionally in a sol form) is, for example, 0.3 to 20% by mass, preferably 0.5 to 10% by mass, and more preferably 1 to 8% by mass. A gel prepared from the liquid mixture with such a solid content is preferred because the gel is capable of maintaining the shape of the angiogenesis device implanted in the body for a long period of time and exhibiting an adhesion preventive ability and has other advantages.

The liquid mixture may be prepared by mixing all the components together. Alternatively, a liquid mixture containing a polymer and optionally a crosslinking agent and a cell culture component as needed (e.g., an aqueous solution of a polyvinyl alcohol resin) may be first prepared, and an angiogenic component may be added to the mixture.

Other components may be added together with or separately from the angiogenic component (e.g., MSCs) and/or the cell culture component.

The aqueous solution containing a polyvinyl alcohol resin may be prepared in any manner, and may be prepared by a conventionally known method for dissolving a PVA, involving, e.g., dispersing a polyvinyl alcohol resin in water at room temperature, agitating the dispersion while raising the temperature to 80°C or more to allow complete dissolution, and cooling the solution.

The crosslinking agent may be in the form of an aqueous solution. The aqueous solution of the crosslinking agent may be prepared in any manner, and may be prepared by, e.g., dispersing the crosslinking agent in water at room temperature, and agitating the dispersion at room temperature to allow dissolution. Alternatively, the aqueous solution of the crosslinking agent may be prepared by dispersing the crosslinking agent in water at room temperature, agitating the dispersion under heating (e.g., at 60°C for 10 minutes) to allow dissolution, and leaving the solution to stand at room temperature.

The aqueous solution of a polyvinyl alcohol resin and the aqueous solution of a crosslinking agent are desirably sterilized by a conventionally known method, such as autoclaving, UV-irradiation, γ-irradiation or filtering. The addition of the angiogenic component under mixing and the subsequent production process of the angiogenesis device are desirably performed under germ-free conditions. The mixture and the angiogenesis device are desirably stored under germ-free conditions.

The aqueous solution of a polyvinyl alcohol resin and the mixture or aqueous solution containing a polyvinyl alcohol resin and a crosslinking agent and optionally MSCs and/or a cell culture component as needed used in the preparation of the angiogenesis device may be left to stand for a certain period of time.

The temperature at which the mixture or the aqueous solution is allowed to stand may be a temperature suitable for storage of the angiogenic component (e.g., MSCs).

The duration during which the mixture or the aqueous solution is allowed to stand to prepare a gel (time for gelation or gelation time) may be selected as appropriate for the concentration of the polymer (a polyvinyl alcohol resin etc.), the amount of the crosslinking agent, the temperature at which the mixture or the aqueous solution is allowed to stand, or other factors, but is usually about 1 hour to about one week at normal temperature. The duration during which the mixture or the aqueous solution is allowed to stand is preferably one hour or more so that the angiogenesis device does not easily break when placed in the body.

When a modified PVA resin is used for the preparation of the angiogenesis device, a pH buffer solution or the like may be added to a liquid mixture containing a modified PVA resin and a crosslinking agent and optionally MSCs and/or a cell culture component as needed to adjust the pH of the liquid mixture and control the gelation time. When the pH of the system is lowered, the gelation time tends to be shortened. When the pH of the system is raised, the gelation time tends to be extended.

An example of a production process of the angiogenesis device using a supporting substrate will be described below.

A liquid polymer mixture (e.g., an aqueous solution or an aqueous gel containing a polyvinyl alcohol resin) containing a cell culture component, optionally in a gel form, is placed on a base material or a base plate (e.g., a glass slide). A supporting substrate, such as a PET mesh (e.g., trade name: PET mesh sheet (TN120), Sanplatec Corp.) is staked on the polymer mixture. A mixture or solution or dispersion or suspension containing an angiogenic component (e.g., MSCs) dissolved or dispersed or suspended in a liquid polymer mixture (e.g., an aqueous solution or an aqueous gel containing a polyvinyl alcohol resin), optionally in a gel form, is spread over the supporting substrate using a gel loading tip. Another supporting substrate (e.g., a PET mesh) is stacked on the polymer mixture-loaded PET mesh in such a manner that the polymer mixture is sandwiched between the supporting substrates. A liquid polymer mixture (e.g., an aqueous solution or an aqueous gel containing a polyvinyl alcohol resin) containing a cell culture component, optionally in a gel form, is placed on the supporting substrate (e.g., a PET mesh). Another base material or base plate (e.g., a glass slide) is stacked on the supporting substrate. The base materials or the base plates (e.g., glass slides) are removed from the assembled polymer mixture and the supporting substrates to give an angiogenesis device according to an aspect of the present invention.

Applications etc.

The device of the present invention can be used to induce or promote angiogenesis.

In particular, the device of the present invention can be implanted or placed in the body of an animal, including a human, to induce angiogenesis at the site in which the angiogenesis device has been implanted or placed.

The implantation site may be, for example, but is not limited to, a subcutaneous site, a subfascial site, the surface of an organ (e.g., the surface of the liver, spleen or other organs), an intraperitoneal site (e.g., inside the greater omentum), the peritoneal cavity, etc. The implantation site is preferably a subcutaneous site.

Subcutaneous sites usually have few blood vessels, but the device of the present invention is capable of efficiently inducing angiogenesis even at such a site with few blood vessels.

The angiogenesis device may be implanted or placed in any manner and may be implanted or placed in accordance with a conventional or known method. For example, instruments known to be used for implantation may be employed.

The subject for implantation of the angiogenesis device and/or a cell-embedding device (described in detail later) may be a human or a non-human animal, for example, a mammal such as a dog or a cat. The non-human animal may be a companion animal. A preferred subject is a human.

The duration of placement of the angiogenesis device may vary depending on the implantation site or other factors, but the implanted angiogenesis device is preferably left at the implantation site for, for example, one week or more, more preferably for 10 days to two months, and particularly preferably for 10 days to one month.

When the angiogenesis device is left in the implantation site for such a duration, a sufficient angiogenesis effect is easily achieved. In particular, both of a sufficient angiogenesis effect and a preventive or inhibitory effect on bleeding, inflammation and/or release of exudates are easily and efficiently achieved.

The implanted angiogenesis device is usually retrieved from the implantation site after a predetermined duration of placement has past. The present invention also includes such a method for retrieving the angiogenesis device.

The retrieval of the angiogenesis device is usually performed without causing bleeding, inflammation or rupture of existing blood vessels or newly formed blood vessels. If bleeding, inflammation or rupture of blood vessels occurs, the blood or exudates may accumulate at the site from which the device has been retrieved.

The angiogenesis device according to the present invention (e.g., the angiogenesis device comprising at least one selected from the polyvinyl alcohol resins (A1) to (A3) as described above) implanted into a site for a selected duration of placement optionally under other conditions rarely forms adhesions to the implantation site, and may efficiently be retrieved from the implantation site without causing bleeding, inflammation or rupture of blood vessels.

An implantation pocket is formed at the site in which the angiogenesis device has been implanted and subsequently retrieved. A cell-embedding device may then be implanted into the implantation pocket as described later.

The present invention also includes a method for implanting or placing a cell-embedding device.

Implantation of a cell-embedding device may be performed in parallel to the implantation or placement of the angiogenesis device or may be performed at the same time of the implantation or placement of the angiogenesis device. Alternatively, the implantation of a cell-embedding device may not be performed in parallel to the implantation or placement of the angiogenesis device or may not be performed at the same time of the implantation or placement of the angiogenesis device.

When the implantation of a cell-embedding device is performed in parallel to the implantation of the angiogenesis device, the angiogenesis device may be implanted near or at the periphery of the cell-embedding device.

A cell-embedding device may be implanted into or placed at the site in which the angiogenesis device has been implanted and subsequently retrieved, i.e., at the implantation site or in the implantation pocket.

In other words, the angiogenesis device of the present invention may be used in combination with a cell-embedding device, or in particular, may be used to induce angiogenesis at a site for implantation of a cell-embedding device or to induce angiogenesis before implantation of a cell-embedding device.

The angiogenesis device of the present invention is capable of inducing angiogenesis at a site for implantation of a cell-embedding device as described above, thereby allowing the cell-embedding device to efficiently exhibit its functions.

In this manner, situations can be avoided where live cells or tissue is implanted before angiogenesis proceeds, and central necrosis may be efficiently inhibited or prevented, thereby allowing the cell-embedding device to exhibit its functions. Replacement of the implanted cell-embedding device is also easily performed when it is needed.

The angiogenesis device according to an aspect of the present invention rarely forms adhesions to the surrounding tissue, and may be retrieved without causing bleeding, inflammation or rupture of blood vessels as described above. After the angiogenesis device is retrieved, a cell-containing device (a cell-embedding device) may be implanted into the site from which the angiogenesis device has been retrieved, thereby much more efficiently facilitating the functions of the cell-containing device (the cell-embedding device).

Implantation of a cell-embedding device into such a site from which the angiogenesis device has been implanted and subsequently retrieved is understood as meaning that the cell-embedding device is efficiently implanted into a site without bleeding, inflammation or rupture of blood vessels.

The inventors studied and found that if the retrieval of the angiogenesis device is accompanied by bleeding, inflammation and/or rupture of blood vessels, blood and/or exudates may easily accumulate between an implanted cell-embedding device and the newly formed vascular bed. The present invention allows a cell-embedding device to be implanted or placed without being accompanied by bleeding, inflammation or rupture of blood vessels. In this manner, newly formed blood vessels may be effectively utilized for the cell-embedding device and may allow the cell-embedding device to efficiently exhibit its functions.

Bleeding, inflammation and/or rupture of blood vessels, if occurs, at the site for implantation of a cell-embedding device can be visually observed, for example, before and after the retrieval or harvest of the angiogenesis device or before the implantation of the cell-embedding device.

The cell-containing device or the cell-embedding device as described above may be cells or tissue itself, or embedded cells or tissue (may also be called the cell-embedding device below) .

The cell-embedding device may be any cell-embedding device and may be, for example, but is not limited to, the cell-embedding device or the like described in Patent literature 1 or 2. The cell-embedding device will be described in detail below.

A biological composition (e.g., cells or tissue) contained, e.g., embedded or encapsulated, in the cell-embedding device is not particularly limited and may be selected as appropriate for the purpose of use of the cell-embedding device.

The biological composition may be, for example, differentiated cells, stem cells or other types of cells derived from the ectoderm, mesoderm or endoderm.

The differentiated cells may be, for example, epidermal cells, smooth muscle cells, osteocytes, bone marrow cells, chondrocytes, skeletal myoblasts, pancreatic parenchymal cells, pancreatic islet cells, pancreatic endocrine cells, pancreatic exocrine cells, pancreatic ductal cells, liver cells (e.g., hepatocytes), thyroid cells, parathyroid cells, adrenal cells, pituitary cells, splenic cells, pinealocytes, renal cells (nephrocytes), spleen cells, anterior pituitary cells, somatotropic cells, dopamine-producing cells, blood cells, cardiac muscle cells, skeletal muscle cells, osteoblasts, nerve cells, pigment cells, adipocytes, etc. These cells may be cells isolated from a living body or may be cells differentiated from stem cells as described later.

The stem cells (e.g., iPS cells) and other types of cells that can be induced to differentiate may be implanted as it is into a living body, or incorporated in the cell-embedding device and implanted in a living body and then induced to differentiate in the living body. Alternatively, differentiated cells are implanted into a living body or embedded in the cell-embedding device.

The stem cells may be, but are not limited to, tissue-resident stem cells, such as epidermal stem cells, hair follicle stem cells, pancreatic stem cells, pancreatic progenitor cells, liver stem cells, neural stem cells, retinal stem cells, and hematopoietic stem cells; embryonic stem cells (ES cells); induced pluripotent stem cells (iPS cells); etc.

These cells are preferably from a mammal, such as a human, a monkey, a pig, a rat, a mouse, a dog, or cat, and are preferably capable of producing and/or secreting a physiologically active substance, such as a hormone or a protein useful for a living body, such as a patient. The type of cells to be implanted can be selected depending on the type of disease in the recipient, such as a patient, to undergo the implantation. When the cells are non-human cells, the cells may carry a human gene introduced thereinto for therapeutic purposes. Examples of the hormone useful for a living body include insulin, thyroid-stimulating hormone, thyroid hormones, parathyroid hormone, growth hormone, thyroxine, glucocorticoids, glucagon, estradiol, and testosterone. Specific examples of the protein useful for a living body include blood coagulation factors, complements, albumin, globulin, and various enzymes, such as metabolic enzymes or digestive enzymes, such as amylase, protease and lipase. Examples of other physiologically active substances include neurotransmitters, such as dopamine.

Specifically, the cells are more preferably pancreatic cells, in particular pancreatic islet cells, or hepatocytes, dopamine-producing cells, or stem cells or progenitor cells thereof, and are further preferably pancreatic cells, in particular pancreatic islet cells, or pancreatic progenitor cells or pancreatic stem cells.

The biological composition contained in the cell-embedding device may be cells or living tissue established for laboratory use, or cells isolated from living tissue. The biological composition is preferably differentiated non-dividing cells. The isolation may be performed by any isolation method in accordance with a conventionally known method. The cells isolated from living tissue are desirably subjected to removal of pathogens, such as pathogenic viruses.

The amount of the biological composition contained in the cell-embedding device may be adjusted as appropriate for the type of biological composition.

The dosage is determined by a physician considering the age, sex and symptoms of the patient, side effects, and other factors, and is not limited to a specific amount. Typically, about one to ten devices may be implanted into an adult. For example, a diabetic patient may receive the cell-embedding device containing usually 1,000 to 1,000,000 IEQ/kg body weight islets, preferably 5,000 to 400,000 IEQ/kg body weight islets, and more preferably 10,000 to 20,000 IEQ/kg body weight islets (IEQ: international unit of the number of pancreatic islets; one IEQ is defined as the volume of a single islet of 150 $\mu$m in diameter) .

The cell-embedding device may be in any shape but is preferably in the same shape as that of the angiogenesis device. The cell-embedding device may be in the shape of a disk, a sphere, a cylinder, or an ellipsoid, or in other shapes, but is preferably in a disk shape.

The cell-embedding device, in particular the device element thereof, may be made of any material, and may be made of a macromolecule (polymer), a metal, a ceramics, etc.

The cell-embedding device may have, for example, an immunoisolation layer containing a polymer, e.g., the polyvinyl alcohol resin (A). In other words, the cell-embedding device may have an immunoisolation layer made of a polymer (a polymer in a gel form) or may have an immunoisolation function exhibited by a polymer (a polymer in a gel form).

The immunoisolation layer or the immunoisolation function refers to a layer or function that prevents permeation of, for example, immune-related proteins, such as antibodies, complements and leucocytes, while allowing permeation of, for example, glucose; hormones such as insulin, thyroid-stimulating hormone, thyroid hormones, parathyroid hormone, growth hormone, thyroxine, glucocorticoids, glucagon, estradiol, and testosterone; proteins such as blood coagulation factors, albumin, globulin, and various enzymes (metabolic enzymes or digestive enzymes such as amylase, protease, and lipase); neurotransmitters, such as dopamine; etc.

The cell-embedding device may be, for example, a bioartificial organ etc.

The cell-embedding device may contain the biological composition in the same manner as in the above angiogenesis device. For example, the cell-embedding device may contain a polymer. The polymer may contain a crosslinking agent and/or a cell culture component. The polymer may be in the form of a gel or an aqueous gel, and the gel may have a predetermined strength as described above.

A preferred embodiment of the cell-embedding device is the same as that of the angiogenesis device. The polymer suitable for the cell-embedding device may be, for example, a polyvinyl alcohol resin, in particular, at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

The resins (A1) and (A2) are suitable as a polymer used to form the angiogenesis device (and the scaffold etc.) as described above, and these polymers are unexpectedly also suitable as a polymer used to form the cell-embedding device or the immunoisolation layer as described in Patent literatures 1 and 2, even though the application of the cell-embedding device is completely different from that of the angiogenesis device.

The cell-embedding device may further contain a supporting substrate as described above.

The present invention also includes an implantation method, in particular, a method for implanting the cell-embedding device, as described above. In the implantation method, the cell-embedding device is implanted into the site from which the angiogenesis device has been implanted and subsequently retrieved as described above. According to such an implanting method, various diseases or symptoms can be prevented or improved depending on the biological composition (cells or tissue) used. The present invention thus also includes a method for preventing and/or treating or improving a disease or a symptom.

Such a method includes implanting the cell-embedding device after the retrieval of the angiogenesis device, and may, of course, include implanting or placing the angiogenesis device before the implantation of the cell-embedding device (angiogenesis step).

Examples of the disease or symptom include, for example, endocrine diseases, such as thyroid diseases, parathyroid diseases, adrenal diseases, pituitary diseases, and pineal diseases; metabolic diseases, such as ornithine transcarbamylase deficiency, hyperammonemia, hypercholesterolemia, homocystinuria, glycogenosis, Crigler-Najjar syndrome, and Wilson's disease; diabetes mellitus, such as type 1 diabetes mellitus, type 2 diabetes mellitus, and pancreatic diabetes; neurodegenerative diseases, such as Parkinson's disease, Alzheimer's disease, amyotrophic lateral sclerosis, and spinocerebellar degeneration; hemophilia; bone diseases such as osteoporosis; cancers such as leukemia; etc.

The duration of the implantation or placement of the cell-embedding device may be, for example, but is not limited to, 10 days or more, one month or more, two months or more, three months or more, six months or more, one year or more, etc.

The angiogenesis device of the present invention is suitable for used in combination with the cell-embedding device as described above. The present invention thus includes a combination device (or a kit, a device or a combination) comprising the angiogenesis device and the cell-embedding device.

EXAMPLES

The present invention will be described in more detail with reference to Examples, but is not limited thereto. Various modifications may be made by a person having ordinary skill in the art, without departing from the technical idea of the present invention. The terms "part" and "%" in the following Examples refer to "parts by mass" and "% by mass", respectively, unless otherwise specified.

Analysis of modified PVA resins

The viscosity of a 4% aqueous solution of a modified PVA resin and the degree of saponification of a modified PVA resin were determined in accordance with JIS K 6726 (1994).

The amount of diacetone acrylamide units, i.e., the degree of modification was determined from the integral value of the peaks attributed to the units in $^1$H-NMR analysis using DMSO-$d_6$ as the solvent.

Analysis of highly syndiotactic PVA resins

The syndiotacticity and the degree of saponification were determined from the integral value of the peaks attributed to the hydroxy groups in $^1$H-NMR analysis using DMSO-$d_6$ as the solvent.

The triad syndiotacticity (%) was determined from the ratio of the three peaks of the protons of the hydroxy groups from 4 to 5 ppm (the three peaks are attributed to isotacticity [I], heterotacticity [H] and syndiotacticity [S] from high to low magnetic field) calculated by the following formula.

$$\text{Triad syndiotacticity (\%)} = [S] / ([I]+[H]+[S]) \times 100$$

The degree of polymerization was determined in terms of the degree of polymerization of polyvinyl acetate in a benzene solution at 30°C in accordance with JIS K 6726 (1977).

Analysis of completely saponified PVA resins

The degree of polymerization and the degree of saponification were determined in accordance with JIS K 6726 (1994).

Analysis of polyacrylic acid hydrazide

The weight average molecular weight of polyacrylic acid hydrazide was determined by size exclusion chromatography under the following conditions.

Measurement conditions:

Solvent: a 50 mM aqueous solution of sodium dihydrogen phosphate
Polymer concentration: 1 mg/mL
Flow rate: 1.0 mL/min
Column temperature: 40°C
Column: Shodex OHPak SB-803HQ, Shodex OHPak SB-805HQ
Standard: pullulan
Detector: RI

The degree of hydrazidation was determined by back titration of $I_2$ using a sodium thiosulfate standard solution. The details of the experimental procedures were as follows.

Experimental procedures:

1. An $I_2$/MeOH solution was prepared.
2. The $I_2$/MeOH solution was titrated using a 0.1 M sodium thiosulfate standard solution (the concentration was determined to be 0.047 M in this measurement).
3. A polymer sample was precisely weighed and dissolved in 20 mL of ion exchanged water.
4. The 0.047 M $I_2$/MeOH solution was added to 2.0 mL of the solution prepared in step 3.
5. Back titration of $I_2$ was performed using the 0.1 M sodium thiosulfate standard solution.

Analysis of aqueous gels

The stress sustained by an aqueous gel was measured with EZ Test EZ-SX (Shimadzu Corporation) following the manufacturer's instructions. Specifically, an aqueous gel of 34 mm in diameter and 17 mm in height was subjected to measurement using a cylindrical jig of 20 mm in diameter to determine the stress sustained by the gel when it was compressed by 20% at 20°C.

The volume of a gel was measured by a caliper, and the gel was immersed in 100 mL of pure water in a plastic container and placed in a 37°C incubator for one month. The gel was removed from the pure water and the volume of the gel was measured again by a caliper. The rate of volume change of the gel was determined from the measured values by the following formula:

$$\text{Rate of volume change (\%)} = \text{volume after immersion (mm}^3\text{)/volume before immersion (mm}^3\text{)} \times 100$$

Evaluation of angiogenesis device

Angiogenesis was induced by implanting an angiogenesis device into a subcutaneous site in rats, and a pancreatic islet device was then implanted into the same site. The angiogenesis device was evaluated by relating its effectiveness to the performance of the functions of the pancreatic islet device.

In particular, a pancreatic islet device was implanted into streptozotocin-induced diabetic rats, and the blood glucose levels were measured over time to evaluate the therapeutic effect. The pancreatic islet device was determined to exhibit a good therapeutic effect when the blood glucose levels were 200 mg/dL or less (diabetes mellitus was healed). The pancreatic islet device was determined to exhibit a poor therapeutic effect when the blood glucose levels were higher than 200 mg/dL (the rats still suffered from diabetes mellitus conditions).

Preparation of modified PVA resins

Synthetic Example 1

Into a flask equipped with an agitator, a thermometer and a dropping-funnel with a reflux condenser were placed 2,000 parts of vinyl acetate, 143 parts of methanol and 3.7 parts of diacetone acrylamide. The air in the system was replaced with nitrogen, and the internal temperature was raised to 60°C. A solution of 0.16 parts of 2,2-azobis(isobutyronitrile) in 100 parts of methanol was added to initiate polymerization. Immediately after the initiation of polymerization, a solution of 70.1 parts of diacetone acrylamide in 46.7 parts of methanol was added dropwise at a constant rate while allowing nitrogen to flow through the flask. At 210 minutes after the initiation of polymerization, m-dinitrobenzene was added as a polymerization terminator to stop the polymerization. The yield at the end of polymerization was 47.1%. Methanol vapor was added to the resulting reaction mixture to distill off the remaining vinyl acetate to give a 35% solution of a diacetone acrylamide-vinyl acetate copolymer in methanol. To 500 parts of this solution, 70 parts of methanol, 1 part of ion exchanged water and 29.3 parts of a 4% solution of sodium hydroxide in methanol were added, and the mixture was thoroughly agitated at 45°C to allow a saponification reaction to proceed. The resulting gelatinous material was pulverized, washed thoroughly with methanol and dried to give D-PVA 1. A 4% aqueous solution of D-PVA 1 had a viscosity of 53.4 mPa·s. D-PVA 1 had a degree of saponification of 98.4 mol% and contained 3.4 mol% diacetone acrylamide units.

Synthetic Examples 2 to 4

D-PVAs 2 to 4 shown in Table 1 below were prepared in the same manner as in Synthetic Example 1 except that the amounts of vinyl acetate and diacetone acrylamide fed to the flask for polymerization were changed and that the polymerization conditions including the amounts of methanol and the polymerization initiator and the saponification conditions were changed.

The diacetone acrylamide-modified PVA may be synthesized by a conventional synthetic method (e.g., the synthetic method described in JP 2015-78324 A).

Table 1

| | | Viscosity of 4% aqueous solution (mPa·s) | Degree of modification (mol%) | Degree of saponification (mol%) |
|---|---|---|---|---|
| Synthetic Example 1 | D-PVA 1 | 53.4 | 3.4 | 98.4 |
| Synthetic Example 2 | D-PVA 2 | 6.0 | 8.6 | 98.4 |
| Synthetic Example 3 | D-PVA 3 | 21.9 | 3.7 | 98.7 |
| Synthetic Example 4 | D-PVA 4 | 154.5 | 5.3 | 98.6 |

Preparation of highly syndiotactic PVA resins (HS-PVAs)

Synthetic Example 5

Into a flask equipped with an agitator, a thermometer, and a dropping-funnel with a reflux condenser were placed 800 parts of vinyl pivalate and 190 parts of methanol. The air in the system was replaced with nitrogen, and the jacket was heated to 80°C to reflux. A solution of 0.07 parts of 2,2-azobis(2,4-dimethylvaleronitrile) in 10 parts of methanol was added to initiate polymerization. At 6 hours after the initiation of polymerization, m-dinitrobenzene was added as a polymerization terminator to stop the polymerization. The polymerization yield was 58%.

After removal of methanol and vinyl pivalate from the resulting reaction mixture, acetone was added to dissolve polyvinyl pivalate to give a 40% solution in acetone.

To 250 parts of the 40% solution in acetone, 110 parts of a 25% solution of potassium hydroxide in methanol was added, and the mixture was thoroughly agitated at 50°C for 2 hours to allow a saponification reaction to proceed. The resulting gelatinous material was pulverized, and 150 parts of acetone and 340 parts of a 25% solution of potassium hydroxide in methanol were added to allow a saponification reaction to proceed at 50°C for 4 hours. The reaction mixture was neutralized with acetic acid, and subjected to solid-liquid separation, washing with methanol and water, and drying to give 30 g of HS-PVA 1.

The degree of polymerization of HS-PVA 1 before saponification was 1450, and the degree of saponification was 99.9 mol%. The triad syndiotacticity of HS-PVA 1 was 37.1%.

Synthetic Example 6

HS-PVA 2 was prepared in the same manner as in Synthetic Example 5 except that the amount of vinyl pivalate was changed from 800 parts to 685 parts, and that 115 parts of vinyl acetate was further added. The molar ratio of vinyl pivalate to vinyl acetate was 80:20. The degree of polymerization of HS-PVA 2 before saponification was 1510, and the degree of saponification was 99.9 mol%. The triad syndiotacticity of HS-PVA 2 was 35.7%.

Synthetic Example 7

Into a flask equipped with an agitator, a thermometer, and a dropping-funnel with a reflux condenser were placed 2,000 parts of vinyl pivalate and 38 parts of methanol. The air in the system was replaced with nitrogen, and the jacket was heated to 80°C to reflux. A solution of 0.03 parts of 2,2-azobis(2,4-dimethylvaleronitrile) in 2 parts of methanol was added to initiate polymerization. At 3 hours after the initiation of polymerization, m-dinitrobenzene was added as a polymerization terminator to stop the polymerization. The polymerization yield was 17%.

After removal of methanol and vinyl pivalate from the resulting reaction mixture, acetone was added to dissolve polyvinyl pivalate to give a 25% solution in acetone.

To 200 parts of the 25% solution in acetone, 54 parts of a 25% solution of potassium hydroxide in methanol was added, and the mixture was thoroughly agitated at 50°C for 2 hours to allow a saponification reaction to proceed. The resulting gelatinous material was pulverized, and 150 parts of acetone and 168 parts of a 25% solution of potassium hydroxide in methanol were added to allow a saponification reaction to proceed at 50°C for 4 hours. The reaction mixture was neutralized with acetic acid, and subjected to solid-liquid separation, washing with methanol and water, and drying to give 17 g of HS-PVA 3.

The degree of polymerization of HS-PVA 3 before saponification was 4440, and the degree of saponification was 99.8 mol%. The triad syndiotacticity of HS-PVA 3 was 36.7%.

Synthetic Example 8

HS-PVA 4 was prepared in the same manner as in Synthetic Example 7 except that the amount of vinyl pivalate was changed from 2,000 parts to 1,600 parts, and that 268 parts of vinyl acetate was further added. The molar ratio of vinyl pivalate to vinyl acetate was 80:20. The degree of polymerization of HS-PVA 4 before saponification was 4530, and the degree of saponification was 99.9 mol%. The triad syndiotacticity of HS-PVA 4 was 35.5%.

The physical property values of the highly syndiotactic PVA resins prepared in Synthetic Examples 5 to 8 are summarized in Table 2 below.

Table 2

|  |  | Degree of polymerization | Degree of saponification (mol%) | Syndiotacticity (%) |
| --- | --- | --- | --- | --- |
| Synthetic Example 5 | HS-PVA 1 | 1450 | 99.9 | 37.1 |
| Synthetic Example 6 | HS-PVA 2 | 1510 | 99.9 | 35.7 |
| Synthetic Example 7 | HS-PVA 3 | 4440 | 99.8 | 36.7 |
| Synthetic Example 8 | HS-PVA 4 | 4530 | 99.9 | 35.5 |

Preparation of polyacrylic acid hydrazides (APAs)

Synthetic Example 9

To an aqueous solution of 20 parts of polyacrylamide with a weight average molecular weight of about 40,000 in 40 parts of ion exchanged water was added 16 parts of hydrazine monohydrate, and the mixture was reacted at 80°C for 15 hours. Ethanol was added to the liquid mixture, and the precipitate was filtered, washed and dried to give APA 1. APA 1 had a weight average molecular weight of about 53,000 and a degree of hydrazidation of 88%.

Synthetic Example 10

APA 2 was prepared by performing a reaction in the same manner as in Synthetic Example 12 except that polyacrylamide with a weight average molecular weight of about 15,000 was used. APA 2 had a weight average molecular weight of about 22,000 and a degree of hydrazidation of 80%.

Synthetic Example 11

APA 3 was prepared by performing a reaction in the same manner as in Synthetic Example 12 except that polyacrylamide with a weight average molecular weight of about 65,000 was used. APA 3 had a weight average molecular weight of about 88,000 and a degree of hydrazidation of 90%.

The physical property values of the polyacrylic acid hydrazides prepared in Synthetic Examples 9 to 11 are summarized in Table 3 below.

Table 3

| | | Degree of hydrazidation (mol%) | Weight average molecular weight |
|---|---|---|---|
| Synthetic Example 9 | APA 1 | 88 | 53000 |
| Synthetic Example 10 | APA 2 | 80 | 22000 |
| Synthetic Example 11 | APA 3 | 90 | 88000 |

Preparation of adipose-derived stem cells (ADSCs)

The subcutaneous adipose tissue was harvested from the abdominal and inguinal regions of 7- to 8-week-old male Lewis rats (Japan SLC) and washed with phosphate buffer solution (PBS).

The adipose tissue was minced, and enzymatically treated in Hanks buffer solution containing 1 mg/mL Type II collagenase (Sigma-Aldrich) and 1 mmol/L $CaCl_2 \cdot 2H_2O$ under shaking at 100 rpm at 37°C for 20 minutes. Remaining undigested adipose tissue was further treated with the enzyme for 20 minutes. The cell and tissue suspension was filtered through a nylon mesh of 70 $\mu$m to remove the residual tissue fragments. The filtrate was centrifuged at 350 $\times$ g at 20°C for 5 minutes to fractionate into a precipitate fraction containing stem cells and a mature adipocyte fraction mainly composed of lipids.

The precipitate fraction was washed with Hanks buffer solution, and suspended in serum-containing medium for adipose-derived stem cells (ADSC-1, Kohjin Bio) containing 100 units/mL penicillin-100 $\mu$g/mL streptomycin (Thermo Fisher Scientific) at 5 $\times$ $10^6$ cells/mL. Ten milliliters of the suspension was transferred to a T75 flask (culture area: 75 $cm^2$), and primary culture was performed under 5% $CO_2$ at a humidity of 95% at 37°C.

The medium was replaced on days 1, 2 and 4 of culture, and non-adherent cells were removed. Cells in the flask were cultured until reaching 50 to 80% confluency. The primary cells were detached from the flask, dissociated into single cells and stored by freezing. The dissociation into single cells was performed by removing the medium from the flask, washing the cells with PBS, adding 3 mL of 0.05% Trypsin-EDTA (Thermo Fisher Scientific) and incubating at 37°C for 3 minutes to detach the cells from the surface of the flask. The isolated adipose-derived stem cells (ADSCs) were suspended in cryopreservative medium CELLBANKER 1plus (Nippon Zenyaku Kogyo) at 1 $\times$ $10^6$ cells/mL, and frozen at -80°C and stored in liquid nitrogen. Frozen cells were then thawed, and suspended in serum-containing medium for ADSCs containing 100 units/mL penicillin-100 $\mu$g/mL streptomycin at 3.3 $\times$ $10^4$ cells/mL.

Ten milliliters of the suspension was transferred to a T75 flask, and the cells were cultured under 5% $CO_2$ at a humidity of 95% at 37°C until reaching 50 to 80% confluency. The second passage cells were detached from the flask, and dissociated into single cells (in the same manner as in the trypsin-EDTA treatment before storing by freezing). The cells were used to produce an adipose-derived stem cell (ADSC) -containing device.

Example 1

Preparation of angiogenesis device

A mixture was prepared by combining 8.0 g of a 6.25% aqueous solution of D-PVA 1 (the polymer prepared in Synthetic Example 1) with 1.0 mL of a 5.0% aqueous solution of APA 1 (the crosslinking agent prepared in Synthetic Example 9 (polyacrylic acid hydrazide)) and 1.0 mL of $10 \times$ Hanks buffer solution. The liquid mixture was heated at 37°C to induce gelation until spinnability was observed. The resulting gel was used to prepare an angiogenesis device.

The gel was poured into a silicone mold (32 mm in diameter and 1 mm in thickness) and left to stand at 37°C for about 10 minutes. A PET mesh (30 mm in diameter) was placed on the gel, and a mixture of ADSCs ($5 \times 10^5$ cells) and the gel was uniformly spread over the surface of the mesh and left to stand at 37°C for about 10 minutes . Another PET mesh was placed on the mixture, and the mold was filled with the gel. Gelation was induced under 5% $CO_2$ at 4°C for 16 hours.

The concentration of D-PVA 1 in the gel (hydrogel) was 5.0%, and the concentration of APA 1 in the gel was 0.5%. The stress sustained by the gel prepared in accordance with the formulation was 5.2 kPa. The rate of volume change of the gel was about 50%. The density of ADSCs in the gel was $7.0 \times 10^2$ cells/mm$^2$.

Implantation and placement of angiogenesis device

The angiogenesis device was placed into a subcutaneous site in rats for four weeks. Before placement of the device, the subcutaneous tissue had no bleeding or release of exudates and had few newly formed blood vessels that were barely visible to the naked eye. After placement of the angiogenesis device for four weeks, newly formed blood vessels in the subcutaneous tissue were clearly visible to the naked eye.

The number of newly formed blood vessels was apparently increased at the site in which the angiogenesis device was placed, whereas adhesions between the surface of the device and the subcutaneous tissue were rarely observed and the device was very easily detached from the subcutaneous tissue. No bleeding or release of exudates was observed in the subcutaneous site when the device was retrieved.

Due to this, after retrieval of the device, implantation of another cell, tissue or device was easily carried out on the site (e.g., the pancreatic islet-embedding device as described below was easily inserted into the site).

The angiogenesis device after four weeks of placement was reduced in volume to about 50%.

The implantation or placement of the device was performed on five rats, and the rats showed similar results, i.e., the rats showed sufficient angiogenesis and no bleeding or release of exudates when the device was retrieved.

Preparation of pancreatic islet cells for pancreatic islet-embedding device

Pancreatic islets were isolated from 11- to 14-week-old male Lewis rats (Japan SLC). Cold Hanks' balanced salt solution (HBSS) containing 0.8 mg/mL collagenase type V (Sigma-Aldrich) was injected through the common bile duct into the pancreas of the rats to digest the pancreas at 37°C for 12 minutes to separate pancreatic islets from the pancreatic tissue. Concentration gradient centrifugation was performed using Histopaque-1119 (Sigma-Aldrich) and Lymphoprep (Axis-Shield, Norway) to collect the pancreatic islets. The pancreatic islets were cultured in RPMI 1640 medium containing 5.5 mmol/L glucose and 10% fetal bovine serum (FBS) under 5% $CO_2$ at 37°C overnight.

Preparation of aqueous solution of modified PVA resin and crosslinking agent in a sol state

To a 25 mL tube were added 8.0 g of a 6.25% aqueous solution of D-PVA 1 prepared in Synthetic Example 1 and 1.0 mL of $10 \times$ HBSS (Hanks' balanced salt solution), and the mixture was agitated by shaking the tube up and down.

The mixture was spin down in a centrifuge (trade name: Hybrid high-speed refrigerated centrifuge 6200, Kubota Corporation), and the mixture was left to stand at 37°C for 10 minutes. Then, 1.0 mL of a 5% aqueous solution of APA 1 prepared in Synthetic Example 9 was added as a crosslinking agent, and the tube was shaken up and down 15 times. The mixture was spin down in the centrifuge, and the tube was again shaken up and down 15 times.

The mixture was centrifuged at 3,000 rpm at 25°C for 1 minute and left to stand at 37°C. The viscosity of the aqueous solution in a sol state was checked at appropriate intervals until the sol beaded up within 3 to 4 seconds, indicating that the sol was in a state optimal for embedding pancreatic islets. The tube was taken out of the hot bath, and left to stand on ice for 1 minute. The tube was centrifuged at 3,000 rpm at 25°C for 1 minute to give a sol containing the modified PVA resin in an amount of 5% and the crosslinking agent in an amount of 0.5%.

The concentration of D-PVA 1 in the gel (hydrogel) was 5.0%, and the concentration of APA 1 in the gel was 0.5%. The stress sustained by the gel prepared in accordance with the formulation was 5.2 kPa.

Preparation of pancreatic islet-embedding device

An amount of 160 µL of the sol prepared on a dish was placed on a glass slide. A PET mesh (trade name: PET mesh sheet (TN120), Sanplatec Corp.) was placed on the sol. The culture medium components were removed from the prepared pancreatic islet cells as thoroughly as possible, and the cells were suspended in 50 µL of an aliquot of the sol to prepare a suspension. The suspension of the pancreatic islet cells was spread over the PET mesh. Another PET mesh was further stacked on the sol-loaded PET mesh in such a manner that the suspension of the pancreatic islet cells was sandwiched between the PET meshes. On the PET meshes, 140 µL of the sol was placed. Another glass slide was then mounted on the sol. The assembled sol and PET meshes were placed in a moist chamber, and left to stand at 4°C for 48 hours to prepare a pancreatic islet-embedding device (in the form of an aqueous gel).

Storing of pancreatic islet-embedding device

The pancreatic islet-embedding device assembled as above was taken off the glass slides, immersed in 5 mL/well of a preservation medium (RPMI 1640 medium containing 10% FBS and glucose adjusted to a concentration of 5.5mM) in a 6-well plate, and stored at 4°C for about 16 hours.

Implantation

Rats were treated with induction of angiogenesis and received streptozotocin injection to induce diabetes mellitus. The pancreatic islet-embedding device (in the form of an aqueous gel) stored in the above described manner was implanted and placed into a subcutaneous site in the rats.

Evaluation of healing of diabetes mellitus

After implantation of the pancreatic islet-embedding device, the blood glucose levels were measured over time to examine the therapeutic effect. The pancreatic islet-embedding device was determined to exhibit a good therapeutic effect when the blood glucose levels were 200 mg/dL or less (diabetes mellitus was healed). The pancreatic islet-embedding device was determined to exhibit a poor therapeutic effect when the blood glucose levels were higher than 200 mg/dL (the rats still suffered from diabetes mellitus conditions).

Comparative Example 1

The pancreatic islet-embedding device (in the form of an aqueous gel) was implanted into the streptozotocin-induced diabetic rats to evaluate the therapeutic effect on diabetes mellitus in the same manner as in Example 1 except that implantation and placement of the angiogenesis device was not performed.
The evaluation of the therapeutic effect on diabetes mellitus was performed on five rats.
The results of the evaluation of the therapeutic effect on diabetes mellitus in Example 1 and Comparative Example 1 are summarized in Table 4 below. The term "blood glucose levels before implantation" refers to the blood glucose levels measured just before the pancreatic islet device was implanted after the retrieval of the angiogenesis device in Example 1.

Table 4

| | Rat No. | Pancreatic islet device | | |
|---|---|---|---|---|
| | | Blood glucose levels before implantation (mg/dL) | Blood glucose levels 60 days after implantation (mg/dL) | Therapeutic effect |
| Example 1 | 1 | 497 | 103 | Good |
| | 2 | 499 | 109 | Good |
| | 3 | 501 | 65 | Good |
| | 4 | 501 | 126 | Good |
| | 5 | 501 | 169 | Good |

(continued)

| | Rat No. | Pancreatic islet device | | |
| --- | --- | --- | --- | --- |
| | | Blood glucose levels before implantation (mg/dL) | Blood glucose levels 60 days after implantation (mg/dL) | Therapeutic effect |
| Comparative Example 1 | 1 | 501 | 501 | Poor |
| | 2 | 501 | 485 | Poor |
| | 3 | 501 | 459 | Poor |
| | 4 | 501 | 501 | Poor |
| | 5 | 477 | 476 | Poor |

As apparent from the results, the angiogenesis device of Example 1 induced sufficient angiogenesis in the subcutaneous site.

The angiogenesis device of Example 1 not only induced sufficient angiogenesis but also caused no bleeding or release of exudates in the subcutaneous site when the device was retrieved, thereby promoting efficient implantation of another device and allowing the implanted device to efficiently exhibit or achieve its functions (the therapeutic effect on diabetes mellitus in Example 1).

The inventors found that sufficient angiogenesis can be induced by a conventional angiogenesis device but when retrieved, bleeding and release of exudates can be observed. The inventors also found that even when a pancreatic islet device is implanted after retrieval of a conventional angiogenesis device in the same manner as above, a sufficient therapeutic effect on diabetes mellitus is not demonstrated in evaluation experiments conducted in the same manner as above.

These findings revealed that prevention of bleeding and/or release of exudates at the time of retrieval of an angiogenesis device is also important for allowing another implanted device to exhibit sufficient functions.

Preparation of other gels

The device prepared using the aqueous gel containing the polymer of the Synthetic Example effectively functioned as an angiogenesis device, as described above. Based on the results, aqueous gels serving as such a device were prepared.

Example 2

Preparation of aqueous gel containing modified PVA resin and measurement of stress sustained by the gel

A mixture was prepared by combining 16.0 g of a 6.25% aqueous solution of D-PVA 1 prepared in Synthetic Example 1, 2.0 mL of a 10% aqueous solution of APA 2 prepared in Synthetic Example 10, and 2.0 mL of 10 × HBSS (Hanks' balanced salt solution). The mixture was poured into a cylindrical container of 34 mm in diameter and left to stand at 37°C for 30 minutes. The mixture was left to stand at 4°C for further 48 hours to give an aqueous gel of 34 mm in diameter and 17 mm in height.

The concentration of the PVA in the gel was 5.0%, and the concentration of the crosslinking agent was 0.5%. The stress sustained by the gel as measured at 20°C was 5.3 kPa. The rate of volume change was 45.5%.

Examples 3 to 17

Aqueous gels were prepared in the same manner as in Example 1 except that the types and concentrations of the modified PVA resin and/or the crosslinking agent, the gelation time and other factors were changed as appropriate as shown in Table 5 below. The stress sustained by the gels and the rate of volume change were determined. To the aqueous gels, 10 × HBSS was added so that the concentration of HBSS in the gels was 10% by mass.

In the table below, the term "ADH" indicates adipic acid dihydrazide.

Table 5

| Example | Modified PVA resin | Crosslinking agent | Concentration in gel (mass%) | | Gelation time | | Stress (kPa) | Rate of volume change (%) |
|---|---|---|---|---|---|---|---|---|
| | | | Modified PVA resin | Crosslinking agent | 37°C | 4°C | | |
| 2 | D-PVA 1 | APA 2 | 5.0 | 0.50 | 30 min | 48 hr | 5.3 | 45.5 |
| 3 | D-PVA 1 | APA 2 | 5.0 | 0.50 | 100 min | 48 hr | 5.4 | 45.5 |
| 4 | D-PVA 1 | APA 2 | 5.0 | 0.50 | 180 min | 48 hr | 5.6 | 49.0 |
| 5 | D-PVA 1 | APA 2 | 5.0 | 0.50 | 24 hr | - | 5.5 | 60.3 |
| 6 | D-PVA 1 | APA 2 | 3.0 | 0.50 | 100 min | 48 hr | 1.1 | 45.5 |
| 7 | D-PVA 1 | APA 2 | 3.0 | 0.50 | 210 min | 48 hr | 1.1 | 45.5 |
| 8 | D-PVA 1 | APA 2 | 3.0 | 0.50 | 24 hr | - | 1.1 | 45.5 |
| 9 | D-PVA 4 | APA 2 | 6.0 | 1.00 | 120 min | 48 hr | 44.0 | 69.2 |
| 10 | D-PVA 4 | APA 2 | 6.0 | 0.75 | 120 min | 48 hr | 18.2 | 60.3 |
| 11 | D-PVA 4 | APA 2 | 7.5 | 0.75 | 120 min | 48 hr | 44.1 | 69.2 |
| 12 | D-PVA 2 | ADH | 5.0 | 0.50 | 100 min | 48 hr | 2.2 | 45.5 |
| 13 | D-PVA 3 | APA 2 | 5.0 | 0.50 | 140 min | 48 hr | 6.0 | 49.0 |
| 14 | D-PVA 3 | APA 2 | 3.0 | 0.50 | 120 min | 48 hr | 1.2 | 45.5 |
| 15 | D-PVA 3 | APA 2 | 10.0 | 0.50 | 180 min | 48 hr | 38.9 | 69.2 |
| 16 | D-PVA 2 | APA 2 | 10.0 | 0.25 | 120 min | 48 hr | 5.8 | 60.3 |
| 17 | D-PVA 2 | APA 2 | 10.0 | 0.15 | 120 min | 48 hr | 2.1 | 60.3 |

Example 18

Preparation of aqueous gel containing highly syndiotactic PVA resin and measurement of stress sustained by the gel

HS-PVA 1 of Synthetic Example 6 was dissolved in ion exchanged water in a closed container at 160°C, and cooled to 90°C to prepare a 6.25% aqueous solution of HS-PVA 1. Then, 16.0 g of the aqueous solution was cooled to 40°C, and 2.0 mL of 10 × HBSS (Hanks' balanced salt solution) and 2.0 mL of ion exchanged water were added and mixed well. The resulting sol was poured into a cylindrical container of 34 mm in diameter, and left to stand at 4°C for 3 hours to allow gelation of the sol to give an aqueous gel of 34 mm in diameter and 17 mm in height.

The concentration of the PVA in the aqueous solution was 5.0%. The stress sustained by the gel as measured at 20°C was 0.8 kPa. The rate of volume change was 60.3%.

Examples 19 to 24

Aqueous gels were prepared in the same manner as in Example 18 except that the type and concentration of the highly syndiotactic PVA resin, the gelation time and other factors were changed as appropriate as shown in Table 6 below. The stress sustained by the gels and the rate of volume change were determined. To the aqueous gels, 10 × HBSS was added so that the concentration of HBSS in the gels was 10% by mass.

Table 6

| Example | Highly syndiotactic PVA resin | Concentration in gel (%) | Gelation time | Stress (kPa) | Rate of volume change (%) |
|---|---|---|---|---|---|
| | | Highly syndiotactic PVA resin | 4°C | | |
| 18 | HS-PVA 1 | 5.0 | 3 hr | 0.8 | 60.3 |
| 19 | HS-PVA 1 | 5.0 | 90 hr | 1.2 | 60.3 |
| 20 | HS-PVA 1 | 7.0 | 3 hr | 2.0 | 69.2 |
| 21 | HS-PVA 2 | 5.0 | 3 hr | 0.7 | 45.5 |
| 22 | HS-PVA 3 | 5.0 | 3 hr | 1.2 | 49.0 |
| 23 | HS-PVA 3 | 10.0 | 45 hr | 8.5 | 69.2 |
| 24 | HS-PVA 4 | 9.0 | 43 hr | 6.4 | 64.7 |

Example 25

Preparation of aqueous gel containing completely saponified PVA resin and measurement of stress sustained by the gel

To 16.0 g of a 12.5% aqueous solution of a completely saponified PVA resin 1 (the degree of polymerization: 1700, the degree of saponification: 99.4 mol%) were added 2.0 mL of 10 × HBSS (Hanks' balanced salt solution) and 2.0 mL of ion exchanged water, and the mixture was mixed well. The aqueous solution was frozen at -20°C for 5 hours and thawed at 4°C for 19 hours. This freezing and thawing cycle was repeated three times to give an aqueous gel.
The concentration of the PVA in the aqueous solution was 10.0%. The stress sustained by the gel as measured at 20°C was 7.2 kPa. The rate of volume change was 69.2%.

Examples 26 to 30

Aqueous gels were prepared in the same manner as in Example 23 except that the type and concentration of the completely saponified PVA resin, the number of freezing and thawing cycles and other factors were changed as appropriate as shown in Table 7 below. The stress sustained by the gels and the rate of volume change were determined. To the aqueous gels, 10 × HBSS was added so that the concentration of HBSS in the gels was 10% by mass.

Table 7

| Example | Completely saponified PVA resin | Degree of polymerization | Degree of saponification (mol%) | Concentration in gel (%) | Freezing and thawing cycles | Stress (kPa) | Rate of volume change (%) |
|---|---|---|---|---|---|---|---|
| | | | | PVA resin | | | |
| 25 | PVA 1 | 1700 | 99.4 | 10.0 | 3 cycles | 7.2 | 69.2 |
| 26 | PVA 1 | 1700 | 99.4 | 10.0 | 1 cycles | 1.9 | 60.3 |
| 27 | PVA 2 | 1000 | 98.5 | 10.0 | 3 cycles | 1.4 | 49.0 |
| 28 | PVA 2 | 1000 | 98.5 | 10.0 | 1 cycles | 0.3 | 45.5 |
| 29 | PVA 3 | 5000 | 99.5 | 5.0 | 3 cycles | 3.4 | 69.2 |
| 30 | PVA 3 | 5000 | 99.5 | 5.0 | 1 cycles | 1.2 | 60.3 |

Examples 31 to 34

Aqueous gels were prepared in the same manner as in Example 1 or 18 except that the type and concentration of PVA resin (the modified PVA resin or the highly syndiotactic PVA resin), the type of crosslinking agent, the gelation time and other factors were changed as appropriate as shown in Table 8 below. The stress sustained by the gels and the rate of volume change were determined. To the aqueous gels, 10 × HBSS was added so that the concentration of HBSS in the gels was 10% by mass.

Table 8

| Example | PVA resin | Crosslinking agent | Concentration in gel (mass%) | | Gelation time | | Stress (kPa) | Rate of volume change (%) |
| --- | --- | --- | --- | --- | --- | --- | --- | --- |
| | | | PVA resin | Crosslinking agent | 37°C | 4°C | | |
| 31 | D-PVA 3 | APA 1 | 5.0 | 0.50 | 130 min | 48 hr | 6.7 | 50.1 |
| 32 | D-PVA 4 | APA 1 | 4.0 | 0.50 | 120 min | 48 hr | 6.4 | 48.3 |
| 33 | HS-PVA 1 | - | 5.9 | - | - | 3 hr | 1.5 | 64.6 |
| 34 | HS-PVA 4 | - | 7.0 | - | - | 3 hr | 1.6 | 54.3 |

The gels prepared in the above Examples were used to produce angiogenesis devices in the same manner as in Example 1, and placement (implantation) of the angiogenesis devices were performed in the same manner as in Example 1. As with the results of Example 1, angiogenesis was observed. The angiogenesis devices were easily retrieved, and no bleeding or release of exudates was observed. The induction of angiogenesis by the angiogenesis devices allowed another implanted device to efficiently exhibit or achieve its functions.

The representative results of the Examples will be described below. In particular, the therapeutic effect on diabetes mellitus was evaluated in the same manner as in Example 1 except that the devices prepared in Examples 31 to 34 were used in place of the angiogenesis device of Example 1. The results are shown in Table 9 below.

The therapeutic effect on diabetes mellitus was assessed on only one rat because the five rats in Example 1 demonstrated similar results or showed similar tendency.

Table 9

| | Pancreatic islet device | | |
| --- | --- | --- | --- |
| | Blood glucose levels before implantation (mg/dL) | Blood glucose levels 60 days after implantation (mg/dL) | Therapeutic effect |
| Example 31 | 470 | 66 | Good |
| Example 32 | 491 | 41 | Good |
| Example 33 | 430 | 69 | Good |
| Example 34 | 423 | 111 | Good |

INDUSTRIAL APPLICABILITY

The present invention provides an angiogenesis device. The device of the invention is applicable as, for example, an angiogenesis device adapted to allow a separately implanted cell- or tissue-embedding device to exhibit its intrinsic, sufficiently high performance.

**Claims**

1. An angiogenesis device for inducing angiogenesis at a site for implantation of a cell- or tissue-containing device, wherein the angiogenesis device comprises an angiogenic component and a polymer.

2. An angiogenesis device adapted to be used in combination with a cell- or tissue-containing device, wherein the angiogenesis device comprises an angiogenic component and a polymer.

3. A combination device comprising an angiogenesis device and a cell- or tissue-containing device, wherein the angiogenesis device comprises an angiogenic component and a polymer.

4. The device according to any one of claims 1 to 3, wherein the angiogenic component comprises mesenchymal stem cells.

5. The device according to any one of claims 1 to 4, wherein the angiogenic component comprises adipose-derived stem cells.

6. The device according to any one of claims 1 to 5, wherein the polymer comprises a polyvinyl alcohol resin (A).

7. The device according to any one of claims 1 to 6, wherein the polymer comprises at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

8. The device according to any one of claims 1 to 7, wherein the polymer comprises at least one or more polyvinyl alcohol resins (A) selected from a modified polyvinyl alcohol resin having an active carbonyl group (A1), and a polyvinyl alcohol resin having a triad syndiotacticity of 32 to 40% (A2).

9. The device according to claim 7 or 8, wherein the polyvinyl alcohol resin (A1) comprises a diacetone acrylamide-modified polyvinyl alcohol.

10. The device according to claim 9, wherein the diacetone acrylamide-modified polyvinyl alcohol contains 0.5 to 15 mol% diacetone acrylamide units.

11. The device according to any one of claims 7 to 10, wherein the polyvinyl alcohol resin (A2) has a degree of saponification of 90 mol% or more and a degree of polymerization of 100 to 10,000.

12. The device according to any one of claims 7 to 11, wherein the polyvinyl alcohol resin (A2) is a saponified product of a polymer containing vinyl pivalate as a polymerization component, and wherein the polyvinyl alcohol resin (A2) contains vinyl pivalate units.

13. The device according to any one of claims 1 to 12, wherein the polymer is in the form of an aqueous gel.

14. The device according to any one of claims 1 to 13, wherein the polymer is in the form of an aqueous gel with a stress of 0.1 kPa or more at 20°C.

15. The device according to any one of claims 1 to 14, wherein the polymer is in the form of an aqueous gel with a rate of volume change of 10% or more as determined by immersing the gel in water for one month.

16. The device according to any one of claims 1 to 15, wherein the polymer is in the form of an aqueous gel with a stress of 0.3 kPa or more at 20°C and a rate of volume change of 20% or more as determined by immersing the gel in water for one month.

17. The device according to any one of claims 13 to 16, wherein the aqueous gel further comprises a crosslinking agent.

18. The device according to any one of claims 13 to 17, wherein the aqueous gel comprises at least one crosslinking agent selected from hydrazide compounds and semicarbazide compounds.

**19.** The device according to any one of claims 1 to 18, wherein the angiogenesis device further comprises a cell culture component.

**20.** The device according to claim 19, wherein the cell culture component contains at least one buffer solution selected from an acetate buffer solution and a phosphate buffer solution.

**21.** The device according to any one of claims 1 to 20, wherein the angiogenic component is present within the polymer.

**22.** The device according to any one of claims 1 to 21, wherein the angiogenic component is dissolved or dispersed in the polymer.

**23.** The device according to any one of claims 1 to 22, wherein the polymer forms a scaffold and/or an adhesion preventive layer.

**24.** The device according to any one of claims 1 to 23, wherein the device is used for subcutaneous implantation.

**25.** The device according to any one of claims 1 to 24, wherein the cell- or tissue-containing device has an immunoisolation layer comprising the polyvinyl alcohol resin (A).

**26.** The device according to any one of claims 1 to 25, wherein the cell- or tissue-containing device has an immunoisolation layer comprising at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

**27.** An implantation method comprising implanting a cell- or tissue-containing device into a site in which the angiogenesis device according to any one of claims 1 to 26 has been implanted and subsequently retrieved.

**28.** A method for preventing and/or treating a disease or a symptom, the method comprising implanting a cell- or tissue-containing device into a site in which the angiogenesis device according to any one of claims 1 to 26 has been implanted and subsequently retrieved.

**29.** The method according to claim 27 or 28, further comprising implanting the angiogenesis device.

**30.** The method according to any one of claims 27 to 29, wherein the angiogenesis device is retrieved without causing bleeding, inflammation and/or rupture of a blood vessel.

**31.** The method according to any one of claims 27 to 30, wherein the device is implanted subcutaneously.

Fig. 1

# EP 4 101 479 A1

<table>
<tr><td colspan="2" align="center"><strong>INTERNATIONAL SEARCH REPORT</strong></td><td>International application No.<br>PCT/JP2021/003991</td></tr>
</table>

**A. CLASSIFICATION OF SUBJECT MATTER**
A61L 27/14(2006.01)i; A61K 9/06(2006.01)i; A61K 35/12(2015.01)i; A61K 35/28(2015.01)i; A61K 45/00(2006.01)i; A61K 47/30(2006.01)i; A61K 47/32(2006.01)i; A61L 27/16(2006.01)i; A61L 27/38(2006.01)i; A61L 27/40(2006.01)i; A61L 27/52(2006.01)i; A61L 27/54(2006.01)i; A61P 3/00(2006.01)i; A61P 3/10(2006.01)i; A61P 5/00(2006.01)i; A61P 7/04(2006.01)i; A61P 19/08(2006.01)i; A61P 25/28(2006.01)i; A61P 35/00(2006.01)i; A61P 43/00(2006.01)i
FI:    A61L27/14; A61L27/16; A61L27/38; A61L27/40; A61L27/52; A61L27/54; A61K35/28; A61K45/00; A61K47/30; A61K47/32; A61K9/06; A61P3/00; A61P3/10; A61P5/00; A61P7/04; A61P19/08; A61P25/28; A61P35/00; A61P43/00 121; A61K35/12

According to International Patent Classification (IPC) or to both national classification and IPC

**B. FIELDS SEARCHED**

Minimum documentation searched (classification system followed by classification symbols)
A61L27/14; A61K9/06; A61K35/12; A61K35/28; A61K45/00; A61K47/30; A61K47/32; A61L27/16; A61L27/38; A61L27/40; A61L27/52; A61L27/54; A61P3/00; A61P3/10; A61P5/00; A61P7/04; A61P19/08; A61P25/28; A61P35/00; A61P43/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2000-178180 A (KYOTO UNIVERSITY PRESIDENT) 27 June 2000 (2000-06-27) claims, paragraphs [0009], [0015], examples, etc. | 1-3, 6, 13-18, 21-24, 27-31 |
| Y | claims, paragraphs [0009], [0015], examples, etc. | 1-32 |

☒ Further documents are listed in the continuation of Box C.      ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 March 2021 (16.03.2021) | 23 March 2021 (23.03.2021) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

35

**INTERNATIONAL SEARCH REPORT**

International application No.

PCT/JP2021/003991

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2017-86313 A (TOHOKU UNIVERSITY) 25 May 2017 (2017-05-25) claims, paragraphs [0007]-[0009], examples, etc. | 1-3, 13-18, 21-24, 27-31 |
| Y | claims, paragraphs [0007]-[0009], examples, etc. | 1-31 |
| Y | WO 2019/044991 A1 (FUJIFILM CORPORATION) 07 March 2019 (2019-03-07) claims, paragraphs [0029], [0081]-[0084], [0108]-[0109], [0124], [0132], examples, etc. | 1-31 |
| Y | 水井崇浩，外9名，脂肪由来幹細胞を付加したリコンビナントペプチドの導入による新規皮下膵島移植法の開発，第46回日本膵・膵島移植研究会プログラム・抄録集， 2019, p. 61, column AS-06, non-official translation (MIZUI, Takahiro et al., "Development of a novel subcutaneous Islet transplantation method by introducing a recombinant peptide with added adipose-derived stem cells", Programs and abstracts of the 46th Annual Congress of Japanese Pancreas and Islet Transplantation Association) | 1-31 |
| Y | WO 2018/155621 A1 (JAPAN VAM & POVAL CO., LTD.) 30 August 2018 (2018-08-30) claims, paragraphs [0011]-[0012], [0067], [0070], examples, etc. | 1-31 |
| Y | WO 2018/155622 A1 (JAPAN VAM & POVAL CO., LTD.) 30 August 2018 (2018-08-30) claims, paragraphs [0011]-[0012], [0062], [0065], examples, etc. | 1-31 |

Form PCT/ISA/210 (continuation of second sheet) (January 2015)

INTERNATIONAL SEARCH REPORT
Information on patent family members

International application No.

PCT/JP2021/003991

| Patent Documents referred in the Report | Publication Date | Patent Family | Publication Date |
|---|---|---|---|
| JP 2000-178180 A | 27 Jun. 2000 | (Family: none) | |
| JP 2017-86313 A | 25 May 2017 | US 2019/0167572 A1 claims, paragraphs [0007]-[0009], examples WO 2017/078177 A1 | |
| WO 2019/044991 A1 | 07 Mar. 201g | EP 3677289 A1 claims, paragraphs [002g], [0086]-[0089], [0124]-[0128], [0147], [0155], examples CN 111050815 A | |
| WO 2018/155621 A1 | 30 Aug. 2018 | US 2019/0365947 A1 claims, paragraphs [0015]-[0016], [0141], [0146], examples EP 3586824 A1 CN 110418635 A KR 10-2019-0121811 A | |
| WO 2018/155622 A1 | 30 Aug. 2018 | EP 3587555 A1 claims, paragraphs [0014]-[0015], [0127], [0131], examples CN 110418835 A KR 10-2019-0121812 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018155621 A
- WO 2018155622 A

- JP 2015078324 A